# EUROPÄISCHE PATENTSCHRIFT

(11) **EP 1 483 389 B1**
(45) Veröffentlichungstag und Bekanntmachung des Hinweises auf die Patenterteilung: **04.11.2015**
(21) Anmeldenummer: 03714811.1
(22) Anmeldetag: 12.03.2003
(51) Int. Cl.: A61K 39/00, A61K 38/00, G01N 33/574, C07K 16/30, A61K 39/395, A61K 48/00, C07K 14/47, C12Q 1/68

(54) **DIFFERENTIELL IN TUMOREN EXPRIMIERTE GENPRODUKTE UND DEREN VERWENDUNG**
GENETIC PRODUCTS DIFFERENTIALLY EXPRESSED IN TUMORS AND USE THEREOF
PRODUITS GENIQUES D'EXPRESSION DIFFERENCIEE DANS LES TUMEURS ET LEUR UTILISATION

(30) Priorität: 13.03.2002 DE 10211088
(43) Veröffentlichungstag der Anmeldung: 08.12.2004
(60) Teilanmeldung: 10010623.6
(73) Patentinhaber: Ganymed Pharmaceuticals AG, 55131 Mainz (DE)
(72) Erfinder: SAHIN, Ugur, 55116 Mainz (DE); TÜRECI, Özlem, 55116 Mainz (DE); KOSLOWSKI, Michael, 85764 Oberschleißheim (DE)
(74) Vertreter: Schnappauf, Georg
(86) Internationale Anmeldenummer: PCT/EP2003/002556
(87) Internationale Veröffentlichungsnummer: WO 2003/076631

(56) Entgegenhaltungen:
- EP-A- 0 270 056
- WO-A-01/09345
- WO-A-02/10363
- WO-A-02/068579
- CHEN HAIMING ET AL: "A testis-specific gene, TPTE, encodes a putative transmembrane tyrosine phosphatase and maps to the pericentromeric region of human chromosomes 21 and 13, and to chromosomes 15, 22, and Y" HUMAN GENETICS, BERLIN, DE, Bd. 105, Nr. 5, November 1999 (1999-11), Seiten 399-409, XP002194326 ISSN: 0340-6717
- DATABASE SWALL [Online] ID: LDHC_HUMAN 1. August 1988 (1988-08-01), "L-lactate dehydrogenase C chain (EC 1.1.1.27)" XP002256961 accession no. EBI Database accession no. P07864
- SCHEURLE DANIELA ET AL: "Cancer gene discovery using Digital Differential Display" CANCER RESEARCH, Bd. 60, Nr. 15, 1. August 2000 (2000-08-01), Seiten 4037-4043, XP002256957 ISSN: 0008-5472
- MARTELANGE VALERIE ET AL: "Identification on a human sarcoma of two new genes with tumor-specific expression" CANCER RESEARCH, Bd. 60, Nr. 14, 15. Juli 2000 (2000-07-15), Seiten 3848-3855, XP002256958 ISSN: 0008-5472
- LETHE BERNARD ET AL: "LAGE-1, a new gene with tumor specificity" INTERNATIONAL JOURNAL OF CANCER, NEW YORK, NY, US, Bd. 76, Nr. 6, 10. Juni 1998 (1998-06-10), Seiten 903-908, XP000964728 ISSN: 0020-7136
- LUCAS S ET AL: "IDENTIFICATION OF A NEW MAGE GENE WITH TUMOR-SPECIFIC EXPRESSION BY REPRESENTATIONAL DIFFERENCE ANALYSIS" CANCER RESEARCH, AMERICAN ASSOCIATION FOR CANCER RESEARCH, BALTIMORE, MD, US, Bd. 58, Nr. 4, 15. Februar 1998 (1998-02-15), Seiten 743-752, XP001120296 ISSN: 0008-5472
- BERA TAPAN K ET AL: "PATE, a gene expressed in prostate cancer, normal prostate, and testis, identified by a functional genomic approach" PROCEEDINGS OF THE NATIONAL ACADEMY OF SCIENCES OF THE UNITED STATES, Bd. 99, Nr. 5, 5. März 2002 (2002-03-05), Seiten 3058-3063, XP002256959 March 5, 2002 ISSN: 0027-8424
- KOSLOWSKI MICHAEL ET AL: "Multiple splice variants of lactate dehydrogenase C selectively expressed in human cancer" CANCER RESEARCH, Bd. 62, Nr. 22, 15. November 2002 (2002-11-15), Seiten 6750-6755, XP002256960 ISSN: 0008-5472

## Beschreibung

Trotz interdisziplinärer Ansätze und Ausreizung klassischer Therapiemodalitäten gehören Krebserkrankungen weiterhin zu den führenden Todesursachen. Neuere therapeutische Konzepte zielen darauf ab, das patienteneigene Immunsystem durch Einsatz von rekombinanten Tumorvakzinen und anderen spezifischen Maßnahmen wie Antikörpertherapie in das therapeutische Gesamtkonzept mit einzubeziehen. Voraussetzung für den Erfolg einer solchen Strategie ist die Erkennung von Tumor-spezifischen oder Tumor-assoziierten Antigenen bzw. Epitopen durch das Immunsystem des Patienten, dessen Effektorfunktionen interventionell verstärkt werden sollen. Tumorzellen unterscheiden sich biologisch wesentlich von ihren nichtmalignen Ursprungszellen. Diese Differenzen sind durch während der Tumorentwicklung erworbene genetische Veränderungen bedingt und führen u.a. auch zur der Bildung qualitativ oder quantitativ veränderter molekulare Strukturen in den Krebszellen. Werden solche Tumor-assoziierten Strukturen vom spezifischen Immunsystem des tumortragenden Wirtes erkannt, spricht man von tumor-assoziierten Antigenen. An der spezifischen Erkennung von tumor-assoziierten Antigenen sind zelluläre und humorale Mechanismen beteiligt, die zwei miteinander funktionell vernetzte Einheiten darstellen: CD4⁺ und CD8⁺ T-Lymphozyten erkennen prozessierte Antigene, die auf den Molekülen der MHC-(Major Histocompatibility complex = HistokompatibilitätsAntigene) Klassen 11 bzw. I präsentiert werden, während B-Lymphozyten zirkulierende Antikörpermoleküle produzieren, die direkt an unprozessierte Antigene binden. Die potentielle klinisch-therapeutische Bedeutung von tumor-assoziierten Antigenen ergibt sich aus der Tatsache, dass die Erkennung von Antigenen auf neoplastischen Zellen durch das Immunsystem zur Initiierung von cytotoxischen Effektormechanismen führt und bei Vorhandensein von T-Helferzellen die Elimination der Krebszellen bewirken kann (Pardoll, Nat. Med. 4:525-31, 1998). Entsprechend ist es eine zentrale Zielsetzung der Tumorimmunologie, diese Strukturen molekular zu definieren. Die molekulare Natur dieser Antigene blieb lange enigmatisch. Erst als entsprechende Klonierungstechniken entwickelt wurden, gelang es, durch Analyse der Zielstrukturen von cytotoxischen T-Lymphozyten (CTL) (van der Bruggen et al., Science 254:1643-7, 1991) bzw. mit zirkulierenden Autoantikörpern (Sahin et al., Curr. Opin. Immunol. 9:709-16, 1997) als Sonden cDNA-Expressionbanken von Tumoren systematisch auf tumor-assoziierte Antigene zu screenen. Hierzu wurden cDNA-Expressionsbanken aus frischem Tumorgewebe hergestellt und in geeigneten Systemen als Proteine rekombinant exprimiert. Aus Patienten isolierte Immuneffektoren, nämlich CTL-Klone mit Tumorspezifischem Lysemuster, oder zirkulierende Autoantikörper wurden genutzt, um die respektiven Antigene zu klonieren.

Durch diese Ansätze sind in den letzten Jahren eine Vielzahl von Antigenen in verschiedenen Neoplasien definiert worden. Von großem Interesse ist dabei die Klasse der Cancer/Testis-Antigene (CTA). CTA und sie kodierende Gene (Cancer/Testis-Gene oder CTG) sind durch ihr charakteristisches Expressionsmuster definiert [Tureci et al, Mol Med Today. 3:342-9, 1997]. Sie finden sich nicht in Normalgeweben bis auf Testis bzw. Keimzellen, werden jedoch in einer Reihe von humanen Malignomen exprimiert und zwar nicht tumortypspezifisch, sondern mit unterschiedlicher Häufigkeit in Tumorentitäten ganz unterschiedlicher Herkunft (Chen & Old, Cancer J. Sci. Am. 5:16-7, 1999). Auch Serumreaktivitäten gegen CTA finden sich nicht in gesunden Kontrollen, sondern lediglich in Tumorpatienten. Insbesondere aufgrund ihrer Gewebeverteilung ist diese Antigenklasse von besonderem Wert für immuntherapeutische Vorhaben und wird in derzeit laufenden klinischen Patientenstudien getestet (Marchand et al., Int. J. Cancer 80:219-30, 1999; Knuth et al., Cancer Chemother. Pharmacol. 46: S46-51, 2000).

Allerdings nutzen die oben dargestellten klassischen Verfahren zur Antigenidentifizierung Immuneffektoren (zirkulierende Autoantikörper oder CTL-Klone) aus Patienten mit in der Regel bereits fortgeschrittenem Krebs als Sonden. Aus einer Reihe von Daten geht hervor, dass Tumoren z.B. zur Tolerisierung und Anergisierung von T-Zellen führen können und gerade im Verlauf der Erkrankung diejenigen Spezifitäten aus dem Immuneffektorenrepertoire verloren gehen, die eine effektive Immunerkennung bewirken könnten. Aus laufenden Patientenstudien hat sich noch kein gesicherter Beweis für eine tatsächliche Wirkung der bisher entdeckten und genutzten tumor-assoziierten Antigene ergeben. Entsprechend kann nicht ausgeschlossen werden, dass spontane Immunantworten evozierende Proteine die falschen Zielstrukturen sind.

Es war die Aufgabe der vorliegenden Erfindung Zielstrukturen für eine Diagnose von Krebserkrankungen bereitzustellen.

Diese Aufgabe wird erfindungsgemäß durch den Gegenstand der Patentansprüche gelöst.

Erfindungsgemäß wurde eine Strategie für eine Identifizierung und Bereitstellung Tumorassoziiert exprimierter Antigene und der dafür kodierenden Nukleinsäuren verfolgt. Diese Strategie beruht auf der Tatsache, dass eigentlich Testis- und damit Keimzell-spezifische Gene, die normalerweise in adulten Geweben silent sind, in Tumorzellen ektop und unerlaubt reaktiviert werden. Durch Datamining wird zunächst eine möglichst komplette Liste aller bekannten Testis-spezifischen Gene aufgestellt und diese sodann durch Expressionsanalysen mittels spezifischer RT-PCR auf ihre aberrante Aktivierung in Tumoren evaluiert. Datamining ist ein bekanntes Verfahren zur Identifizierung von Tumor-assoziierten Genen. Bei den herkömmlichen Strategien werden allerdings in der Regel Transkriptome von Normalgewebebanken elektronisch von Tumorgewebsbanken subtrahiert unter der Annahme, dass die verbleibenden Gene Tumor-spezifisch sind (Schmitt et al., Nucleic Acids Res. 27:4251-60,1999; Vasmatzis et al., Proc. Natl. Acad. Sci. USA. 95:300-4, 1998. Scheurle et al., Cancer Res. 60:4037-43, 2000).

Das erfindungsgemäße Konzept, das sich als viel erfolgreicher erwiesen hat, beruht jedoch darauf, Datamining zur elektronischen Extraktion aller Testis-spezifischer Gene zu nutzen und diese sodann auf ektope Expression in Tumoren zu evaluieren.

Somit wird vorliegend eine Strategie zur Identifizierung von differentiell in Tumoren exprimierten Genen beschrieben. Diese kombiniert Datamining von öffentlichen Sequenzbanken (*"in silico*") mit darauffolgenden evaluierenden labor-experimentellen ("wet bench") Untersuchungen.

Eine kombinierte Strategie basierend auf zwei unterschiedlichen bioinformatischen Skripten ermöglichte erfindungsgemäß die Identifizierung neuer Mitglieder der Cancer/Testis (CT) Genklasse. Diese sind bisher als rein Testis-, Keimzell-, oder Spermien-spezifisch eingestuft worden. Die Erkenntnis, dass diese Gene aberrant in Tumorzellen aktiviert werden, erlaubt, ihnen eine substantiell neue Qualität mit funktionellen Implikationen zuzuordnen. Die Identifizierung und Bereitstellung dieser tumor-assoziierten Gene und der dadurch kodierten Genprodukte erfolgte erfindungsgemäß unabhängig von einer immunogenen Wirkung.

Die erfindungsgemäß identifizierten Tumor-assoziierten Antigene weisen eine Aminosäuresequenz auf, die von einer Nukleinsäure kodiert wird, die aus der Gruppe ausgewählt ist, bestehend aus (a) einer Nukleinsäure, die eine Nukleinsäuresequenz umfasst, die aus der Gruppe bestehend aus SEQ ID NOs: 19-21 und 54-57 ausgewählt ist und (b) einer Nukleinsäure, die zu der Nukleinsäure unter mindestens 98% Sequenzidentität aufweist. In einer bevorzugten Ausführungsform weist ein erfindungsgemäß identifiziertes Tumor-assoziiertes Antigen eine Aminosäuresequenz auf, die von einer Nukleinsäure kodiert wird, die aus der Gruppe bestehend aus SEQ ID NOs: 19-21 und 54-57 ausgewählt ist. In einer weiteren bevorzugten Ausführungsform umfasst ein erfindungsgemäß identifiziertes tumor-assoziiertes Antigen eine Aminosäuresequenz, die aus der Gruppe bestehend aus SEQ ID NOs: 22-24, 58-61, 81 und 82 ausgewählt ist.

Die vorliegende Erfindung betrifft allgemein die Verwendung von erfindungsgemäß identifizierten Tumor-assoziierten Antigenen oder von Teilen davon, von dafür kodierenden Nukleinsäuren oder von Nukleinsäuren, die gegen die kodierenden Nukleinsäuren gerichtet sind oder von Antikörpern, die gegen die erfindungsgemäß identifizierten Tumor-assoziierten Antigene oder Teile davon gerichtet sind, für die Diagnose von Krebserkrankungen. Diese Nutzung kann einzelne, aber auch Kombinationen von mehreren dieser Antigene, funktionalen Fragmente, Nukleinsäuren, Antikörper etc betreffen, in einer Ausführungsform auch in Kombination mit anderen tumor-assoziierten Genen und Antigenen für eine Diagnose und Verlaufskontrolle.

Bevorzugte Erkrankungen für eine Diagnose sind Krebserkrankungen, bei denen eine selektive Expression des erfindungsgemäß identifizierten Tumor-assoziierten Antigens vorliegt.

Die Erfindung betrifft Nukleinsäuren und Genprodukte, die tumorzellassoziiert exprimiert werden und die durch verändertes Spleißen (Spleißvarianten) bekannter Gene bzw. durch veränderte Translation unter Nutzung alternativer offener Leserahmen entstehen. Diese Nukleinsäuren umfassen die Sequenzen gemäß (SEQ ID NO: 20, 21 und 54-57) des Sequenzprotokolls. Die erfindungsgemäßen Spleißvarianten sind als Targets für die Diagnostik und Therapie von Tumorerkrankungen verwendbar.

Für die Entstehung von Spleißvarianten können verschiedenste Mechanismen ursächlich sein, beispielsweise
- die Nutzung variabler Transkriptionsinitiationsstellen
- die Nutzung zusätzlicher Exons
- vollständiges oder unvollständiges Aus Spleißen von einzelnen oder mehreren Exons,
- über Mutation veränderte Spleißregulatorsequenzen (Deletion bzw. Schaffung neuer Donor/Acceptorsequenzen),
- die unvollständige Elimination von Intronsequenzen.

Das veränderte Spleißen eines Gens führt zu einer veränderten Transkriptsequenz (Spleißvariante). Wird eine Spleißvariante im Bereich ihrer veränderten Sequenz translatiert, resultiert ein verändertes Protein, welches sich von dem ursprünglichen in Struktur und Funktion deutlich unterscheiden kann. Bei tumorassoziierten Spleißvarianten können tumorassoziierte Transkripte und tumorassoziierte Proteine/Antigene entstehen. Diese können als molekulare Marker sowohl zum Nachweis von Tumorzellen als auch zum therapeutischen Targeting von Tumoren genutzt werden. Die Detektion von Tumorzellen z.B. im Blut, Serum, Knochenmark, Sputum, Bronchial-Lavage, Körpersekreten und Gewebsbiopsien kann erfindungsgemäß z.B. nach Extraktion von Nukleinsäuren durch PCR-Amplifikation mit Spleißvariantenspezifischen Oligonukleotiden erfolgen. Zum Nachweis eignen sich erfindungsgemäß alle Sequenz-abhängigen Detektionssysteme. Neben der PCR sind diese z.B. Genchip-/Microarraysysteme, Northern-Blot, RNAse protection assays (RDA) und andere. Allen Detektionssystemen ist gemeinsam, dass die Detektion auf einer spezifischen Hybridisierung mit mindestens einer Spleißvarianten-spezifischen Nukleinsäuresequenz basiert. Die Detektion von Tumorzellen kann jedoch auch erfindungsgemäß durch Antikörper erfolgen, die ein durch die Spleißvariante kodiertes spezifisches Epitop erkennen. Für die Herstellung der Antikörper können Peptide zur Immunisierung verwendet werden, die für diese Spleißvariante spezifisch sind. Für die Immunisierung eignen sich besonders die Aminosäuren, die deutliche Epitopunterschiede zu der Variante(n) des Genprodukts aufweisen, welche(s) bevorzugt in gesunden Zellen gebildet wird. Der Nachweis der Tumorzellen mit Antikörper kann dabei an einer vom Patienten isolierten Probe oder als Imaging mit intravenös applizierten Antikörpern erfolgen. Neben der diagnostischen Nutzbarkeit stellen Spleißvarianten, die neue oder veränderte Epitope aufweisen, attraktive Targets für die Immuntherapie dar. Die erfindungsgemäßen Epitope können zum Targeting von therapeutisch wirksamen monoklonalen Antikörpern oder T-Lymphozyten genutzt werden. Bei der passiven Immuntherapie werden hierbei Antikörper oder T-Lymphozyten adoptiv transferriert, die Spleißvarianten-spezifische Epitope erkennen. Die Generierung von Antikörpern kann wie bei anderen Antigenen auch unter Nutzung von Standardtechnologien (Immunisierung von Tieren, Panningstrategien zur Isolation von rekombinanten Antikörpern) unter Nutzung von Polypeptiden, die diese Epitope beinhalten, erfolgen. Alternativ können zur Immunisierung Nukleinsäuren genutzt werden, die für Oligo- oder Polypeptide kodieren, die diese Epitope beinhalten. Verschiedene Techniken zur in vitro oder in vivo Generierung von epitopspezifischen T-Lymphozyten sind bekannt und ausführlich beschrieben z.B. (Kessler JH, et al. 2001, Sahin et al., 1997) und basieren ebenfalls auf der Nutzung von Oligo-oder Polypeptide, die die Spleißvarianten-spezifischen Epitope beinhalten oder Nukleinsäuren, die für diese kodieren. Oligo-oder Polypeptiden, die die Spleißvariantenspezifischen Epitope beinhalten oder Nukleinsäuren, die für diese Polypeptide kodieren sind auch für die Nutzung als pharmazeutisch wirksame Substanzen bei der aktiven Immuntherapie (Vakzinierung, Vakzintherapie) verwendbar.

In einem Aspekt betriftt die Erfindung die Verwendung
(i) einer Polynukleotid-Sonde, die spezifisch mit einer Nukleinsäure, die für ein Tumor-assoziiertes Antigen kodiert, hybridisiert, und/oder
(ii) eines Antikörpers, der spezifisch an ein Tumor-assoziiertes Antigen bindet, und/oder
(iii) eines Proteins oder Peptids, das spezifisch an einen Antikörper gegen ein Tumor-assoziiertes Antigen bindet,
zur Herstellung einer pharmazeutischen Zusammensetzung zur Diagnose oder Überwachung einer Krebserkrankung, die sich durch die Expression des Tumor-assoziierten Antigens auszeichnet, in einer aus einem Patienten isolierten biologischen Probe, wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, und 54-57 ausgewählt ist,
   und
(b) einer Nukleinsäure, die zu der Nukleinsäure unter (a) mindestens 98% Sequenzidentität aufweist.

In einem Aspekt betrifft die Erfindung die Verwendung eines Antikörpers, der an ein Tumor-assoziiertes Antigen bindet und mit einem diagnostischen Mittel gekoppelt ist, zur Herstellung einer pharmazeutischen Zusammensetzung zur Diagnose oder Überwachung einer Krebserkrankung, die sich durch die Expression des Tumor-assoziierten Antigens auszeichnet, wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, und 54-57 ausgewählt ist,
   und
(b) einer Nukleinsäure, die zu der Nukleinsäure unter (a) mindestens 98% Sequenzidentität aufweist.

In einer erfindungsgemäßen Ausführungsform ist der Antikörper, der an ein Tumor-assoziiertes Antigen bindet, ein monoklonaler, chimärer oder humanisierter Antikörper oder ein Fragment eines Antikörpers.

In einer erfindungsgemäßen Ausführungsform umfasst das Tumor-assoziierte Antigen eine Aminosäuresequenz, die aus der Gruppe bestehend aus SEQ ID NOs: 22-24, 58-61, 81, und 82 ausgewählt ist.

In einem Aspekt betrifft die Erfindung einen Antikörper, der spezifisch an ein Tumorassoziiertes Antigen bindet, wobei das Tumor-assoziierte Antigen von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, und 54-57 ausgewählt ist,
   und
(b) einer Nukleinsäure, die zu der Nukleinsäure unter (a) mindestens 98% Sequenzidentität aufweist.
zur Verwendung in einem Verfahren zur Diagnose einer Krebserkrankung, die sich durch die Expression des Tumor-assoziierten Antigens auszeichnet.

In einer erfindungsgemäßen Ausführungsform umfasst das Protein oder Polypeptid eine Aminosäuresequenz, die aus der Gruppe ausgewählt ist, bestehend aus SEQ ID NOs: 22-24,58-61, 81, und 82.

In einer erfindungsgemäßen Ausführungsform ist der Antikörper ein monoklonaler, chimärer oder humanisierter Antikörper oder ein Fragment eines Antikörpers-ist.

In einer erfindungsgemäßen Ausführungsform ist der Antikörper an ein diagnostisches Mittel gekoppelt.

In einer erfindungsgemäßen Ausfiihrungsform ist das diagnostische Mittel ein Toxin.

In einem Aspekt betrifft die Erfindung die Verwendung eines Kits zur Diagnose einer Krebserkrankung, die sich durch die Expression eines Tumor-assoziierten Antigens auszeichnet, umfassend Mittel zum Nachweis
(i) der Nukleinsäure, die für das Tumor-assoziierte Antigen kodiert,
(ii) des Tumor-assoziierten Antigens,
(iii) von Antikörpern, die an das Tumor-assoziierte Antigen binden, und/oder
(iv) von T-Zellen, die für einen Komplex zwischen dem Tumor-assoziierten Antigen oder einem Teil davon, der mindestens 6 aufeinander folgende Aminosäuren umfasst, und einem MHC-Molekül spezifisch sind,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, und 54-57 ausgewählt ist,
   und
(b) einer Nukleinsäure, die zu der Nukleinsäure unter (a) mindestens 98% Sequenzidentität aufweist.

In einer erfindungsgemäßen Ausführungsform sind die Mittel zum Nachweis der Nukleinsäure, die für das Tumor-assoziierte Antigen kodiert, Nukleinsäuremolektile für die selektive Amplifikation der Nukleinsäure.

In einem Aspekt betrifft die Erfindung die Verwendung eines Antikörpers, der an TPTE bindet und mit einem diagnostischen Mittel gekoppelt ist, zur Herstellung einer pharmazeutischen Zusammensetzung zur Diagnose oder Überwachung eines metastasierenden Tumors, der sich durch die Expression von TPTE auszeichnet, wobei das TPTE eine Sequenz aufweist, die von einer Nukleinsäure kodiert wird, bestehend aus:
(a) einer Nukleinsäure, die die Nukleinsäuresequenz gemäß SEQ ID NO:19 umfasst,
   und
(b) einer Nukleinsäure, die zu der Nukleinsäure unter (a) mindestens 98% Sequenzidentität aufweist.

In einer erfindungsgemäßen Ausführungsform ist der Antikörper ein monoklonaler, chimärer oder humanisierter Antikörper oder ein Fragment eines Antikörpers.

In einem Aspekt betrifft die Erfindung ein *in vitro* Verfahren zur Diagnose einer Krebserkrankung, die sich durch die Expression eines Tumor-assoziierten Antigens auszeichnet, umfassend
(i) den Nachweis einer Nukleinsäure, die für ein Tumor-assoziiertes Antigen kodiert, und/oder
(ii) den Nachweis eines Tumor-assoziierten Antigens und/oder
(iii) den Nachweis eines Antikörpers gegen ein Tumor-assoziiertes Antigen und/oder
(iv) den Nachweis von cytotoxischen oder Helfer-T-Zellen, die für ein Tumor-assoziiertes Antigen oder einen Teil davon, der mindestens 6 aufeinander folgende Aminosäuren umfasst, spezifisch sind, in einer aus einem Patienten isolierten biologischen Probe, wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 19-21, und 54-57 ausgewählt ist,
      und
   (b) einer Nukleinsäure, die zu der Nukleinsäure unter (a) mindestens 98% Sequenzidentität aufweist.

In einer erfindungsgemäßen Ausführungsform umfasst der Nachweis
(i) die Kontaktierung der biologischen Probe mit einem Mittel, das spezifisch an die Nukleinsäure, die für das Tumor-assoziierte Antigen kodiert, an das Tumor-assoziierte Antigen, an den Antikörper oder an die cytotoxischen oder Helfer-T-Zellen bindet, und
(ii) den Nachweis der Komplexbildung zwischen dem Mittel und der Nukleinsäure, dem Tumor-assoziierten Antigen, dem Antikörper oder den cytotoxischen oder Helfer-T-Zellen , wobei der Nachweis vorzugsweise mit dem Nachweis in einer vergleichbaren normalen biologischen Probe verglichen wird.

In einem Aspekt betrifft die Erfindung ein *in vitro* Verfahren zur Bestimmung der Regression, des Verlaufs oder des Ausbruchs einer Krebserkrankung, die sich durch die Expression eines Tumor-assoziierten Antigens auszeichnet, umfassend die Überwachung einer Probe aus einem Patienten, der Krebs aufweist oder in Verdacht steht, an Krebs zu erkranken, in Bezug auf einen oder mehrere Parameter, ausgewählt aus der Gruppe, bestehend aus:
(i) der Menge einer Nukleinsäure, die für das Tumor-assoziiertes Antigen kodiert,
(ii) der Menge des Tumor-assoziierten Antigens,
(iii) der Menge an Antikörpern, die an das Tumor-assoziiertes Antigen binden, und
(iv) der Menge an cytolytischen oder Cytokin-ausschüttenden T-Zellen, die für einen Komplex zwischen dem Tumor-assoziierten Antigen oder einem Teil davon, der mindestens 6 aufeinander folgende Aminosäuren umfasst, und einem MHC-Molekül spezifisch sind, wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 19-21, und 54-57 ausgewählt ist,
      und
   (b) einer Nukleinsäure, die zu der Nukleinsäure unter (a) mindestens 98% Sequenzidentität aufweist.

In einer erfindungsgemäßen Ausführungsform umfasst das Verfahren die Bestimmung des oder der Parameter zu einem ersten Zeitpunkt in einer ersten Probe und zu einem zweiten Zeitpunkt in einer weiteren Probe, wobei durch einen Vergleich der beiden Proben der Verlauf der Erkrankung ermittelt wird.

In einer erfindungsgemäßen Ausführungsform erfolgt der Nachweis der Nukleinsäure oder die Überwachung der Menge der Nukleinsäure mit einer Polynukleotid-Sonde, die spezifisch mit der Nukleinsäure hybridisiert.

In einer erfindungsgemäßen Ausführungsform umfasst die Polynukleotid-Sonde eine Sequenz von 6-50 zusammenhängenden Nukleotiden aus der Nukleinsäure, die für das Tumor-assoziierte Antigen kodiert.

In einer erfindungsgemäßen Ausführungsform erfolgt der Nachweis der Nukleinsäure oder die Überwachung der Menge der Nukleinsäure durch selektive Amplifikation der Nukleinsäure.

In einer erfindungsemäßen Ausführungsform erfolgt der Nachweis des Tumor-assoziierten Antigens oder die Überwachung der Menge des Tumor-assoziierten Antigens mit einem Antikörper, der spezifisch an das Tumor-assoziierte Antigen bindet.

In einer erfindungsgemäßen Ausführungsform erfolgt der Nachweis des Antikörpers oder die Überwachung der Menge an Antikörpern mit einem Protein oder Peptid, das spezifisch an den Antikörper bindet.

In einer erfindungsgemäßen Ausführungsform erfolgt der Nachweis der T-Zellen oder die Überwachung der Menge an T-Zellen mit einer Zelle, die den Komplex zwischen dem Tumor-assoziierten Antigen oder dem Teil davon und einem MHC-Molekül präsentiert.

In einer erfindungsgemäßen Ausführungsform sind die Polynukleotid-Sonde, der Antikörper, das Protein oder Peptid oder die Zelle nachweisbar markiert.

In einer erfindungsgemäßen Ausführungsform ist der nachweisbare Marker ein radioaktiver Marker oder ein Enzymmarker.

In einer erfindungsgemäßen Ausführungsform umfasst die Probe Körperflüssigkeit und/oder Körpergewebe.

In einer erfindungsgemäßen Ausführungsform umfasst das Tumor-assoziierte Antigen eine Aminosäuresequenz, die aus der Gruppe bestehend aus SEQ ID NOs: 22-24, 58-61, 81, und 82 ausgewählt ist.

Eine pharmazeutische Zusammensetzung kann einen pharmazeutisch verträglichen Träger und/oder ein Adjuvans umfassen. Das Adjuvans kann aus Saponin, GM-CSF, CpG-Nukleotiden, RNA, einem Zytokin oder einem Chemokin ausgewählt sein. Eine erfindungsgemäße pharmazeutische Zusammensetzung wird vorzugsweise zur Behandlung einer Erkrankung eingesetzt, die sich durch die selektive Expression oder abnormale Expression eines Tumor-assoziierten Antigens auszeichnet. In einer bevorzugten Ausführungsform ist die Erkrankung Krebs

Des weiteren betrifft die Erfindung die in den Ansprüchen näher definierten Verfahren und Verwendungen zur Diagnose einer Krebserkrankung, die sich durch die Expression eines oder mehrerer Tumor-assoziierter Antigene auszeichnet.

Vorliegend werden Verwendungen und ein Verfahren zur Diagnose einer Krebserkrankung beschrieben, die sich durch die Expression des erfindungsgemäß identifizierten Tumor-assoziierten Antigens auszeichnen. Das Verfahren umfasst den Nachweis (i) einer Nukleinsäure, die für das Tumor-assoziierte Antigen kodiert, oder eines Teils davon und/oder (ii) den Nachweis des Tumor-assoziierten Antigens oder eines Teils davon, und/oder (iii) den Nachweis eines Antikörpers gegen das Tumor-assoziierte Antigen oder einen Teil davon und/oder (iv) den Nachweis von cytotoxischen oder Helfer-T-Lymphozyten, die für das Tumor-assoziierte Antigen oder einen Teil davon spezifisch sind in einer aus einem Patienten isolierten biologischen Probe. In bestimmten Ausführungsformen umfasst der Nachweis (i) die Kontaktierung der biologischen Probe mit einem Mittel, das spezifisch an die Nukleinsäure, die für das Tumor-assoziierte Antigen kodiert, oder den Teil davon, an das Tumor-assoziierte Antigen oder den Teil davon, an den Antikörper oder an cytotoxische oder Helfer-T-Lymphozyten, die für das Tumor-assoziierte Antigen oder Teile davon spezifisch sind, bindet und (ii) den Nachweis der Komplexbildung zwischen dem Mittel und der Nukleinsäure oder dem Teil davon, dem Tumor-assoziierten Antigen oder dem Teil davon, dem Antikörper oder den cytotoxischen oder Helfer-T-Lymphozyten. In einer Ausführungsform zeichnet sich die Erkrankung durch die Expression oder abnormale Expression mehrerer verschiedener Tumor-assoziierter Antigene aus und der Nachweis umfasst einen Nachweis mehrerer Nukleinsäuren, die für die mehreren verschiedenen Tumorassoziierten Antigene kodieren, oder von Teilen davon, den Nachweis der mehreren verschiedenen Tumor-assoziierten Antigene oder von Teilen davon, den Nachweis mehrere Antikörper, die an die mehreren verschiedenen Tumor-assoziierten Antigene oder an Teile davon binden oder den Nachweis mehrerer cytotoxischer oder Helfer-T-Lymphozyten, die für die mehreren verschiedenen Tumor-assoziierten Antigene spezifisch sind. In einer weiteren Ausführungsform wird die isolierte biologische Probe aus dem Patienten mit einer vergleichbaren normalen biologischen Probe verglichen.

Vorliegend wird weiterhin ein Verfahren zur Bestimmung der Regression, des Verlaufs oder des Ausbruchs einer Krebserkrankung beschrieben, die sich durch die Expression des erfindungsgemäß identifizierten Tumor-assoziierten Antigens auszeichnet, umfassend die Überwachung einer Probe aus einem Patienten, der die Erkrankung aufweist oder in Verdacht steht, an der Erkrankung zu erkranken in Bezug auf einen oder mehrere Parameter, die aus der Gruppe ausgewählt sind, bestehend aus (i) der Menge der Nukleinsäure, die für das Tumor-assoziierte Antigen kodiert, oder eines Teil davon, (ii) der Menge des Tumor-assoziierten Antigens oder eines Teils davon, (iii) der Menge an Antikörpern, die an das Tumor-assoziierte Antigen oder einen Teil davon binden, und (iv) der Menge an cytolytischen T-Zellen oder Helfer-T-Zellen, die für einen Komplex zwischen dem Tumor-assoziierten Antigen oder einem Teil davon und einem MHC-Molekül spezifisch sind. Vorzugsweise umfasst das Verfahren die Bestimmung des oder der Parameter zu einem ersten Zeitpunkt in einer ersten Probe und zu einem zweiten Zeitpunkt in einer weiteren Probe, wobei durch einen Vergleich der beiden Proben der Verlauf der Erkrankung ermittelt wird. In bestimmten Ausführungsformen zeichnet sich die Erkrankung durch die Expression oder abnormale Expression mehrerer verschiedener Tumor-assoziierter Antigene aus und die Überwachung umfasst eine Überwachung (i) der Menge mehrerer Nukleinsäuren, die für die mehreren verschiedenen Tumor-assoziierten Antigene kodieren, oder von Teilen davon und/oder (ii) der Menge der mehreren verschiedenen Tumor-assoziierten Antigene oder von Teilen davon und/oder (iii) der Menge mehrerer Antikörper, die an die mehreren verschiedenen Tumor-assoziierten Antigene oder an Teile davon binden, und/oder (iv) der Menge mehrerer cytolytischer T-Zellen oder Helfer-T-Zellen, die für Komplexe zwischen den mehreren verschiedenen Tumor-assoziierten Antigenen oder von Teilen davon und MHC-Molekülen spezifisch sind.

Ein Nachweis einer Nukleinsäure oder eines Teils davon oder eine Überwachung der Menge einer Nukleinsäure oder eines Teils davon kann erfindungsgemäß mit einer Polynukleotid-Sonde erfolgen, die spezifisch mit der Nukleinsäure oder dem Teil davon hybridisiert oder kann durch selektive Amplifikation der Nukleinsäure oder des Teils davon erfolgen. In einer Ausführungsform umfasst die Polynukleotid-Sonde eine Sequenz von 6-50, beispielsweise 10-30, 15-30 und 20-30 zusammenhängenden Nukleotiden aus der Nukleinsäure.

In bestimmten Ausführungsformen liegt das nachzuweisende Tumor-assoziierte Antigen oder der Teil davon intrazellulär oder auf der Zelloberfläche vor. Ein Nachweis eines Tumorassoziierten Antigens oder eines Teils davon oder eine Überwachung der Menge eines Tumor-assoziierten Antigens oder eines Teils davon kann mit einem Antikörper erfolgen, der spezifisch an das Tumor-assoziierte Antigen oder den Teil davon bindet.

In weiteren Ausführungsformen liegt das nachzuweisende Tumor-assoziierte Antigen oder der Teil davon in einem Komplex mit einem MHC-Molekül, insbesondere einem HLA-Molckül vor.

Ein Nachweis eines Antikörpers oder die Überwachung der Menge an Antikörpern kann mit einem Protein oder Peptid erfolgen, das spezifisch an den Antikörper bindet.

Ein Nachweis von cytolytischen T-Zellen oder Helfer-T-Zellen oder die Überwachung der Menge an cytolytischen T-Zellen oder Helfer-T-Zellen, die für Komplexe zwischen einem Antigen oder einem Teil davon und MHC-Molekülen spezifisch sind, kann mit einer Zelle erfolgen, die den Komplex zwischen dem Antigen oder dem Teil davon und einem MHC-Molekül präsentiert.

Die für einen Nachweis oder für eine Überwachung verwendete Polynukleotid-Sonde, der Antikörper, das Protein oder Peptid oder die Zelle sind beispielsweise nachweisbar markiert, wobei beispielsweise der nachweisbare Marker ein radioaktiver Marker oder ein Enzymmarker ist. Der Nachweis von T-Lymphozyten kann zusätzlich erfolgen durch Nachweis ihrer Proliferation, ihrer Zytokinproduktion, sowie ihrer cytotoxischen Aktivität, die ausgelöst wird durch die spezifische Stimulation mit dem. Komplex aus MHC und tumorassoziiertem Antigen oder Teilen davon. Der Nachweis von T-Lymphozyten kann ferner erfolgen durch ein rekombinantes MHC-Molekül oder auch ein Komplex aus mehreren MHC-Molekülen, die beladen sind mit dem jeweiligen immunogenen Fragment aus einem oder mehreren der tumorassoziierten Antigene und durch Kontaktierung des spezifischen T-Zell-Rezeptors die spezifischen T-Lymphozyten identifizieren können.

In einem weiteren Aspekt betrifft die Erfindung allgemein ein Verfahren und verschiedene Verwendungen zur Diagnose oder Überwachung einer Krebserkrankung, die sich durch die Expression des erfindungsgemäß identifizierten Tumor-assoziierten Antigens auszeichnet. Solche Verfahren und Verwendungen können die Verabreichung eines Antikörpers umfassen, der an das Tumor-assoziierte Antigen oder einen Teil davon bindet und mit einem diagnostischen Mittel gekoppelt ist. Der Antikörper kann ein monoklonaler Antikörper sein. In weiteren Ausführungsformen ist der Antikörper ein chimärer oder humanisierter Antikörper oder ein Fragment eines natürlichen Antikörpers.

Des weiteren bezieht sich die Erfindung auf eine Nukleinsäure, die aus der Gruppe ausgewählt ist, bestehend aus (a) einer Nukleinsäure, die eine Nukleinsäuresequenz umfasst, die aus der Gruppe bestehend aus SEQ ID NOs: 20-21 und 54-57 ausgewählt ist und (b) einer Nukleinsäure, die zu der Nukleinsäure unter (a) mindestens 98% Sequenzidentität aufweist. Des weiteren bezieht sich die Erfindung auf eine Nukleinsäure, die für ein Protein oder Polypeptid kodiert, das eine Aminosäuresequenz umfasst, ausgewählt aus der Gruppe bestehend aus SEQ ID NOs: 23-24 und 58-61.

Denkbar ist beispielsweise ein rekombinantes Nukleinsäuremolekül, insbesondere DNA- oder RNA-Molekül, das eine erfindungsgemäße Nukleinsäure umfasst.

Die Erfindung bezieht sich auch auf Wirtszellen, die eine erfindungsgemäße Nukleinsäure oder ein rekombinantes Nukleinsäuremolekül, das eine erfindungsgemäße Nukleinsäure umfasst, enthalten.

Die Wirtszelle kann ferner eine Nukleinsäure umfassen, die für ein HLA-Molekül kodiert. Beispielsweise exprimiert die Wirtszelle das HLA-Molekül endogen. Alternativ exprimiert die Wirtszelle das HLA-Molekül und/oder die erfindungsgemäße Nukleinsäure oder einen Teil davon rekombinant. Beispielsweise ist die Wirtszelle nicht-proliferativ. In einer Ausführungsform ist die Wirtszelle eine Antigen-präsentierende Zelle, beispielsweise eine dendritische Zelle, ein Monozyt oder ein Makrophage.

In einer weiteren Ausführungsform bezieht sich die Erfindung auf Oligonukleotide, die mit einer erfindungsgemäß identifizierten Nukleinsäure hybridisieren und als genetische Sonden verwendet werden können. Nukleinsäuremoleküle in der Form von

Oligonukleotid-Primern oder kompetenten Proben, die mit einer erfindungsgemäß identifizierten Nukleinsäure oder Teilen davon hybridisieren, können zum Auffinden von Nukleinsäuren verwendet werden, die zu der erfindungsgemäß identifizierten Nukleinsäure homolog sind. PCR-Amplifikation, Southern- und Northern-Hybridisierung können zum Auffinden homologer Nukleinsäuren eingesetzt werden. Die Hybridisierung kann unter niedrig-, besser unter mittel- und am besten unter hoch-stringenten Bedingungen erfolgen. Der Begriff "stringente Bedingungen" betrifft erfindungsgemäß Bedingungen, die eine spezifische Hybridisierung zwischen Polynukleotiden erlauben.

In einem weiteren Aspekt bezieht sich die Erfindung auf ein Protein oder Polypeptid, das von einer Nukleinsäure kodiert wird, die aus der Gruppe ausgewählt ist, bestehend aus (a) einer Nukleinsäure, die eine Nukleinsäuresequenz umfasst, die aus der Gruppe bestehend aus SEQ ID NOs: 20-21, 54-57 ausgewählt ist und (b) einer Nukleinsäure, die zu der Nukleinsäure unter (a) mindestens 98% Sequenzidentität aufweist. In einer bevorzugten Ausführungsform bezieht sich die Erfindung auf ein Protein oder Polypeptid, das eine Aminosäuresequenz umfasst, ausgewählt aus der Gruppe bestehend aus SEQ ID NOs: 23-24 und 58-61.

In einem weiteren Aspekt bezieht sich die Erfindung auf ein immunogenes Fragment oder Teil des erfindungsgemäß identifizierten Tumor-assoziierten Antigens. Das Fragment bindet vorzugsweise an einen menschlichen HLA-Rezeptor oder menschlichen Antikörper. Vorzugsweise umfasst ein erfindungsgemäßes Fragment eine Sequenz von mindestens 6, beispielsweise mindestens 8, mindestens 10, mindestens 12, mindestens 15, mindestens 20, mindestens 30 oder mindestens 50 Aminosäuren.

In einem weiteren Aspekt betrifft die Erfindung ein Mittel, das an ein erfindungsgemäß identifiziertes Tumor-assoziiertes Antigen oder an den Teil davon bindet. In einer bevorzugten Ausführungsform ist das Mittel ein Antikörper. In weiteren Ausführungsformen ist der Antikörper ein chimärer, ein humanisierter oder mit kombinatorischen Techniken hergestellte Antikörper oder ein Fragment eines Antikörpers. Des weiteren betrifft die Erfindung einen Antikörper, der selektiv an einen Komplex aus (i) einem erfindungsgemäß identifizierten Tumor-assoziierten Antigen oder einem Teil davon und (ii) einem MHC-Molekül bindet, an das das erfindungsgemäß identifizierte Tumor-assoziierte Antigen oder der Teil davon bindet, wobei der Antiköper nicht alleine an (i) oder (ii) bindet. Ein erfindungsgemäßer Antikörper kann ein monoklonaler Antikörper sein. In weiteren Ausführungsformen ist der Antikörper ein chimärer oder humanisierter Antikörper oder ein Fragment eines natürlichen Antikörpers.

Des weiteren betrifft die Erfindung ein Konjugat zwischen einem erfindungsgemäßen Mittel, das an ein erfindungsgemäß identifiziertes Tumor-assoziiertes Antigen oder an einen Teil davon bindet, oder einem erfindungsgemäßen Antikörper und einem diagnostischen Mittel. In einer Ausführungsform ist das diagnostische Mittel ein Toxin.

In einem weiteren Aspekt bezieht sich die Erfindung auf einen Kit zum Nachweis der Expression des erfindungsgemäß identifizierten Tumorassoziierten Antigens, umfassend Mittel zum Nachweis (i) der Nukleinsäure, die für das Tumor-assoziierte Antigen kodiert, (ii) des Tumor-assoziierten Antigens, (iii) von Antikörpern, die an das Tumor-assoziierte Antigen binden, und/oder (iv) von T-Zellen, die für einen Komplex zwischen dem Tumor-assoziierten Antigen oder einem Teil davon und einem MHC-Molekül spezifisch sind. In einer Ausführungsform sind die Mittel zum Nachweis der Nukleinsäure oder des Teils davon Nukleinsäuremoleküle für die selektive Amplifikation der Nukleinsäure, die insbesondere eine Sequenz von 6-50, insbesondere 10-30, 15-30 und 20-30 zusammenhängenden Nukleotiden aus der Nukleinsäure umfassen.

### Detaillierte Beschreibung der Erfindung

Erfindungsgemäß wird ein Gene beschrieben, das in Tumorzellen selektiv exprimiert oder aberrant exprimiert wird und ein Tumor-assoziiertes Antigene darstellt.

Tumor-assoziierte Antigene sind bevorzugte Zielstrukturen für therapeutische Ansätze. Konzeptionell können die therapeutischen Ansätze auf eine Hemmung der Aktivität des selektiv exprimierten tumor-assoziierten Genproduktes zielen. Dies ist dann sinnvoll, wenn die aberrante respektive selektive Expression funktionell von tumorpathogenetischer Bedeutung ist und ihre Unterbindung mit einer selektiven Schädigung der entsprechenden Zellen einhergeht. Andere therapeutische Konzepte betrachten tumorassoziierte Antigene als Markierungen, die Effektormechanismen mit zellschädigendem Potential selektiv zu Tumorzellen rekrutieren. Hierbei ist die Funktion des Zielmoleküls selbst und seine Rolle bei der Tumorentstehung vollkommen unerheblich.

Mit "Derivat" einer Nukleinsäure ist gemeint, dass einzelne oder multiple Nukleotidsubstitution, -deletion und/oder -addition in der Nukleinsäure vorliegen. Weiterhin umfasst der Begriff "Derivat" auch eine chemische Derivatisierung einer Nukleinsäure an einer Nukleotidbase, am Zucker oder am Phosphat. Der Begriff "Derivat" umfasst auch Nukleinsäuren, die nicht in der Natur vorkommende Nukleotide und Nukleotidanaloga enthalten.

Eine Nukleinsäure ist beispielsweise eine Desoxyribonukleinsäure (DNA) oder Ribonukleinsäure (RNA). Nukleinsäuren umfassen genomische DNA, cDNA, mRNA, rekombinant hergestellte und chemisch synthetisierte Moleküle. Eine Nukleinsäure kann als einzelsträngiges oder doppelsträngiges und lineares oder kovalent kreisförmig geschlossenes Molekül vorliegen.

Die beschriebenen Nukleinsäuren sind beispielsweise isoliert. Der Begriff "isolierte Nukleinsäure" bedeutet, dass die Nukleinsäure (i) *in vitro* amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplex eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989 oder Current Protocols in Molecular Biology, F.M. Ausubel et al., Hrsg., John Wiley & Sons, Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5mM NaH₂PO₄ (pH7), 0,5% SDS, 2mM EDTA). SSC ist 0,15 M Natriumchlorid/ 0,15 M Natriumcitrat, pH 7. Nach der Hybridisierung wird die Membran, auf die die DNA übertragen wurde beispielsweise in 2 x SSC bei Raumtemperatur und sodann in 0,1 - 0,5 x SSC/ 0,1 x SDS bei Temperaturen bis 68°C gewaschen.

Komplementäre Nukleinsäuren weisen zum Beispiel mindestens 40%, insbesondere mindestens 50%, mindestens 60%, mindestens 70%, mindestens 80%, mindestens 90% und vorzugsweise mindestens 95%, mindestens 98 oder mindestens 99% Identität der Nukleotide auf.

Nukleinsäuren, die für Tumor-assoziierte Antigene kodieren, können alleine oder in Kombination mit anderen Nukleinsäuren, insbesondere heterologen Nukleinsäuren, vorliegen. Eine Nukleinsäure kann funktionell in Verbindung mit Expressionskontrollsequenzen oder regulatorischen Sequenzen vorliegen, die in Bezug zu der Nukleinsäure homolog oder heterolog sein können. Eine kodierende Sequenz und eine regulatorische Sequenz sind dann "funktionell" miteinander verbunden, falls sie derart kovalent miteinander verknüpft sind, dass die Expression oder Transkription der kodierenden Sequenz unter der Kontrolle oder unter dem Einfluss der regulatorischen Sequenz steht. Falls die kodierende Sequenz in ein funktionelles Protein translatiert werden soll, führt bei einer funktionellen Verbindung einer regulatorischen Sequenz mit der kodierenden Sequenz eine Induktion der regulatorischen Sequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Protein oder Peptid translatiert zu werden.

Der Begriff "Expressionskontrollsequenz" oder "regulatorische Sequenz" umfasst Promotoren, Enhancer und andere Kontrollelemente, die die Expression eines Gens steuern. Beispielsweise sind die Expressionskontrollsequenzen regulierbar. Die genaue Struktur von regulatorischen Sequenzen kann speziesabhängig oder zelltypusabhängig variieren, umfasst jedoch im allgemeinen 5'-nicht-transkribierte und 5'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht transkribierte Regulationssequenzen eine Promotorregion, die eine Promotorsequenz für eine transkriptionelle Kontrolle des funktionell verbundenen Gens einschließt. Regulatorische Sequenzen können auch Enhancer-Sequenzen oder stromaufwärts gelegene Aktivatorsequenzen umfassen.

Zum einen kann also das hier dargestellt tumorassoziierten Antigene mit beliebigen Expressionskontrollsequenzen und Promotoren kombiniert werden. Zum anderen aber können die Promotoren der hier dargestellten tumor-assoziierten Genprodukte mit beliebigen anderen Genen kombiniert werden. Dies erlaubt, die selektive Aktivität dieser Promotoren zu nutzen.

Des weiteren kann eine Nukleinsäure in Verbindung mit einer anderen Nukleinsäure vorliegen, die für ein Polypeptid kodiert, das eine Sekretion des durch die Nukleinsäure kodierten Proteins oder Polypeptids aus einer Wirtszelle steuert. Auch kann eine Nukleinsäure in Verbindung mit einer anderen Nukleinsäure vorliegen, die für ein Polypeptid kodiert, das eine Verankerung des kodierten Proteins oder Polypeptids auf der Zellmembran der Wirtszelle oder seine Kompartimentalisierung in bestimmte Organellen dieser Zelle herbeiführt.

Ein rekombinantes DNA-Molekül ist beispielsweise ein Vektor, gegebenenfalls mit einem Promotor, der die Expression einer Nukleinsäure, z.B. einer Nukleinsäure, die für eine erfindungsgemäßes Tumor-assoziiertes Antigen kodiert, steuert. Der Begriff "Vektor" wird dabei in seiner allgemeinsten Bedeutung verwendet und umfasst jegliche intermediären Vehikel für eine Nukleinsäure, die es z.B. ermöglichen die Nukleinsäure in prokaryotische und/oder in eukaryotische Zellen einzubringen und gegebenenfalls in ein Genom zu integrieren. Solche Vektoren werden vorzugsweise in der Zelle repliziert und/oder exprimiert. Ein intermediäres Vehikel kann z.B. für den Gebrauch bei der Elektroporation, beim Mikroprojektilbeschuss, bei der liposomalen Verabreichung, beim Transfer mit Hilfe von Agrobakterien oder bei der Insertion über DNA- oder RNA-Viren angepasst sein. Vektoren umfassen Plasmide, Phagemide oder Virusgenome.

Die Nukleinsäuren, die für das erfindungsgemäß identifiziertes Tumor-assoziiertes Antigen kodieren, können für eine Transfektion von Wirtszellen eingesetzt werden. Mit Nukleinsäuren ist dabei sowohl rekombinante DNA wie auch RNA gemeint. Rekombinante RNA kann durch in-vitro-Transkription von einer DNA-Matritze hergestellt werden. Sie kann des weiteren vor Applikation durch stabilisierende Sequenzen, Capping und Poly-Adenylierung modifiziert werden. Der Begriff "Wirtszelle" betrifft jede Zelle, die mit einer exogenen Nukleinsäure transformierbar oder transfizierbar ist. Der Begriff "Wirtszellen" umfasst prokaryontische (z.B. *E. coli*) oder eukaryontische (z.B. dendritische Zellen, B-Zellen, CHO-Zellen, COS-Zellen, K562-Zellen, Hefezellen und Insektenzellen). Denkbar sind Säugerzellen wie Zellen aus Mensch, Maus, Hamster, Schwein, Ziege, Primaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und embryonale Stammzellen. In weiteren Ausführungsformen ist die Wirtszelle eine Antigen-präsentierende Zelle, insbesondere eine dendritische Zelle, Monozyt oder ein Makrophage. Eine Nukleinsäure kann in der Wirtszelle in einer einzigen oder in mehreren Kopien vorliegen und wird in einer Ausführungsform in der Wirtszelle exprimiert.

Der Begriff "Expression" wird vorliegend in seiner allgemeinsten Bedeutung verwendet und umfasst die Produktion von RNA oder von RNA und Protein. Er umfasst auch eine teilweise Expression von Nukleinsäuren. Des weiteren kann die Expression transient oder stabil erfolgen. Bevorzugte Expressionssysteme in Säugerzellen umfassen pcDNA3.1 und pRc/CMV (Invitrogen, Carlsbad, CA), die einen selektierbaren Marker enthalten wie ein Gen, das eine Resistenz gegenüber G418 verleiht (und somit eine Selektion stabil transfizierter Zelllinien ermöglicht) und die Enhancer-Promotor-Sequenzen von Cytomegalovirus (CMV).

In den Fällen der Erfindung, in denen ein HLA-Molekül ein Tumor-assoziiertes Antigen oder einen Teil davon präsentiert, kann ein Expressionsvektor auch eine Nukleinsäuresequenz umfassen, das für das HLA-Molekül kodiert. Die Nukleinsäuresequenz, die für das HLA-Molekül kodiert, kann auf demselben Expressionsvektor wie die Nukleinsäure, die für das Tumor-assoziierte Antigen oder den Teil davon kodiert, vorliegen oder beide Nukleinsäuren können auf verschiedenen Expressionsvektoren vorliegen. Im letzteren Fall können die beiden Expressionsvektoren in eine Zelle cotransfiziert werden. Falls eine Wirtszelle weder das Tumor-assoziierte Antigen oder den Teil davon noch das HLA-Molekül exprimiert, werden beide dafür kodierenden Nukleinsäuren entweder auf demselben Expressionsvektor oder auf verschiedenen Expressionsvektoren in die Zelle transfiziert. Falls die Zelle bereits das HLA-Molekül exprimiert, kann nur die Nukleinsäuresequenz, die für das Tumor-assoziierte Antigen oder den Teil davon kodiert, in die Zelle transfiziert werden.

Denkbar sind auch Kits zur Amplifikation einer Nukleinsäure, die für das erfindungsgemäße Tumor-assoziiertes Antigen kodiert. Solche Kits umfassen beispielsweise ein Paar von Amplifikationsprimern, die an die Nukleinsäure hybridisieren, die für das Tumor-assoziierte Antigen kodiert. Die Primer umfassen vorzugsweise eine Sequenz von 6-50, beispielsweise 10-30, 15-30 und 20-30 zusammenhängenden Nukleotiden aus der Nukleinsäure und sind nicht-überlappend, um die Bildung von Primer-Dimeren zu vermeiden. Einer der Primer wird an einen Strang der Nukleinsäure hybridisieren, die für das Tumor-assoziierte Antigen kodiert, und der andere Primer wird an den komplementären Strang in einer Anordnung hybridisieren, die eine Amplifikation der Nukleinsäure, die für das Tumor-assoziierte Antigen kodiert, erlaubt.

Ein erfindungsgemäß verwendetes Oligonukleotid besteht z.B. aus Ribonukleotiden, Desoxyribonukleotiden oder einer Kombination davon. Dabei sind das 5'-Ende eines Nukleotids und das 3'-Ende eines anderen Nukleotids durch eine Phosphodiesterbindung miteinander verknüpft. Diese Oligonukleotide können in herkömmlicher Weise synthetisiert oder rekombinant produziert werden.

Alternativ ist ein erfindungsgemäßes Oligonukleotid ein "modifiziertes" Oligonukleotid. Dabei kann das Oligonukleotid, um beispielsweise seine Stabilität zu erhöhen, auf verschiedenste Art und Weise modifiziert sein ohne dass seine Fähigkeit, an sein Ziel zu binden, beeinträchtigt wird. Der Begriff "modifiziertes Oligonukleotid" bedeutet ein Oligonukleotid bei dem (i) mindestens zwei seiner Nukleotide durch eine synthetische Internukleosidbindung (d.h. eine Internukleosidbindung, die keine Phosphodiesterbindung ist) miteinander verknüpft sind und/oder (ii) eine chemische Gruppe kovalent mit dem Oligonukleotid verbunden ist, die normalerweise nicht bei Nukleinsäuren auftritt. Bevorzugte synthetische Internukleosidbindungen sind Phosphorothioate, Alkylphosphonate, Phosphorodithioate, Phosphatester, Alkylphosphonothioate, Phosphoramidate, Carbamate, Carbonate, Phosphattriester, Acetamidate, Carboxymethylester und Peptide.

Der Begriff "modifiziertes Oligonukleotid" umfasst auch Oligonukleotide mit einer kovalent modifizierten Base und/oder Zucker. "Modifizierte Oligonukleotide" umfassen beispielsweise Oligonukleotide mit Zuckerresten, die kovalent an organische Gruppen mit einem geringen Molekulargewicht gebunden sind, die keine Hydroxylgruppe an der 3'-Position und keine Phosphatgruppe an der 5'-Position sind. Modifizierte Oligonukleotide können beispielsweise einen 2'-O-alkylierten Riboserest oder einen anderen Zucker anstelle von Ribose wie Arabinose umfassen.

Die beschriebenen Proteine und Polypeptide können isoliert vorliegen. Die Begriffe "isoliertes Protein" oder "isoliertes Polypeptid" bedeuten, dass das Protein oder Polypeptid von seiner natürlichen Umgebung getrennt ist. Ein isoliertes Protein oder Polypeptid kann in einem im wesentlichen aufgereinigten Zustand vorliegen. Der Begriff "im wesentlichen aufgereinigt" bedeutet, dass das Protein oder Polypeptid im wesentlichen frei von anderen Substanzen vorliegt, mit denen es in der Natur oder *in vivo* vorliegt.

Solche Proteine und Polypeptide dienen beispielsweise der Herstellung von Antikörpern und sind in einem immunologischen oder diagnostischen Assay oder als Therapeutika einsetzbar. Die beschriebenen Proteine und Polypeptide können aus biologischen Proben wie Gewebe- oder Zellhomogenaten isoliert werden und können auch rekombinant in einer Vielzahl pro- oder eukaryontischer Expressionssysteme exprimiert werden.

"Derivate" eines Proteins oder Polypeptids oder einer Aminosäuresequenz umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften ersetzt, wie Hydrophobizität, Hydrophilizität, Elektronegativität, Volumen der Seitenkette und ähnliches (konservative Substitution). Konservative Substitutionen betreffen beispielsweise den Austausch einer Aminosäure durch eine andere, nachstehend in derselben Gruppe wie die substituierte Aminosäure aufgeführte Aminosäure:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die oben beschriebenen Aminosäure-Varianten können leicht mit Hilfe von bekannten Peptidsynthesetechniken wie z.B. durch "Solid Phase Synthesis" (Merrifield, 1964) und ähnliche Verfahren oder durch rekombinante DNA-Manipulation hergestellt werden. Techniken, um Substitutionsmutationen an vorbestimmten Stellen in DNA einzubringen, die eine bekannte oder teilweise bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen ist z.B. in Sambrook et. aL (1989) ausführlich beschrieben.

"Derivate" von Proteinen oder Polypeptiden umfassen erfindungsgemäß auch einzelne oder multiple Substitutionen, Deletionen und/oder Additionen jeglicher Moleküle, die mit dem Enzym assoziiert sind, wie Kohlenhydrate, Lipide und/oder Proteine oder Polypeptide. Ferner erstreckt sich der Begriff "Derivat" auch auf alle funktionellen chemischen Äquivalente der Proteine oder Polypeptide.

Ein Teil oder Fragment eines Tumor-assoziierten Antigens weist oftmals eine funktionelle Eigenschaft des Polypeptids auf, aus dem es abgeleitet sind. Solche funktionellen Eigenschaften umfassen die Interaktion mit Antikörpern, die Interaktion mit anderen Polypeptiden oder Proteinen, die selektive Bindung von Nukleinsäuren und eine enzymatische Aktivität. Eine bedeutende Eigenschaft ist die Fähigkeit, einen Komplex mit HLA einzugehen und gegebenenfalls eine Immunreaktion zu erzeugen. Diese Immunreaktion kann auf Stimulation von cytotoxischen oder Helfer T-Zellen beruhen. Vorzugsweise umfasst ein erfindungsgemäßer Teil oder Fragment eines Tumor-assoziierten Antigens eine Sequenz von mindestens 6, beispielsweise mindestens 8, mindestens 10, mindestens 12, mindestens 15, mindestens 20, mindestens 30 oder mindestens 50 aufeinanderfolgenden Aminosäuren aus dem Tumor-assoziierten Antigen.

Ein Teil oder ein Fragment einer Nukleinsäure, die für ein Tumor-assoziiertes Antigen kodiert, betrifft erfindungsgemäß den Teil der Nukleinsäure, der zumindest für das Tumor-assoziierte Antigen kodiert und/oder für einen Teil oder ein Fragment des Tumor-assoziierten Antigens wie vorstehend definiert kodiert.

Die Isolierung und Identifizierung des erfindungsgemäßen Gens, das für das Tumor-assoziierte Antigene TPTE kodiert, ermöglicht auch die Diagnose einer Krebserkrankung, die sich durch die Expression von einem oder mehreren Tumor-assoziierten Antigenen auszeichnet. Diese Verfahren bzw. Verwendungen umfassen die Bestimmung einer oder mehrerer Nukleinsäuren, die für ein Tumor-assoziiertes Antigen kodieren, und/oder die Bestimmung der kodierten Tumor-assoziierten Antigene und/oder von davon abgeleiteten Peptiden. Eine Bestimmung der Nukleinsäure kann in herkömmlicher Weise erfolgen, einschließlich durch Polymerase-Kettenreaktion oder Hybridisierung mit einer markierten Sonde. Eine Bestimmung von Tumor-assoziierten Antigenen oder davon abgeleiteten Peptiden kann durch ein Screening von Patienten-Antiseren in Bezug auf eine Erkennung des Antigens und/oder der Peptide erfolgen. Sie kann auch erfolgen durch ein Screening von T-Zellen des Patienten auf Spezifität für das entsprechende tumor-assoziierte Antigen.

Erfindungsgemäß umfasst sind Bindestoffe wie z.B. Antikörper oder Antikörperfragmente, die die Fähigkeit aufweisen, selektiv an das erfindungsgemäße Tumor-assoziierte Antigene zu binden. Antikörper umfassen polyklonate und monoklonale Antikörper, die in herkömmlicher Weise hergestellt werden.

Es ist bekannt, dass nur ein kleiner Teil eines Antikörpermoleküls, das Paratop, an der Bindung des Antikörpers an sein Epitop beteiligt ist (vgl. Clark, W.R (1986), The Experimental Foundations of Modern Immunology, Wiley & Sons, Inc., New York; Roitt, I. (1991), Essential Immunology, 7. Auflage, Blackwell Scientific Publications, Oxford). Die pFc'- und Fc-Regionen sind z.B. Effektoren der Komplementkaskade, sind jedoch nicht an der Antigenbindung beteiligt. Ein Antikörper, von dem die pFc'-Region enzymatisch abgespalten wurde oder der ohne die pFc'-Region hergestellt wurde, bezeichnet als F(ab')₂-Fragment, trägt beide Antigenbindestellen eines vollständigen Antikörpers. In ähnlicher Weise trägt ein Antikörper, von dem die Fc-Region enzymatisch abgespalten wurde oder der ohne die Fc-Region hergestellt wurde, bezeichnet als Fab-Fragment, eine Antigenbindestelle eines intakten Antikörpermoleküls. Des weiteren bestehen Fab-Fragmente aus einer kovalent gebundenen leichten Kette eines Antikörpers und einem Teil der schweren Kette des Antikörpers, bezeichnet als Fd. Die Fd-Fragmente sind die Haupt-Determinanten der Antikörper-Spezifität (ein einzelnes Fd-Fragment kann mit bis zu zehn verschiedenen leichten Ketten assoziiert werden, ohne die Spezifität des Antikörpers zu verändern) und Fd-Fragmente behalten bei einer Isolierung die Fähigkeit, an ein Epitop zu binden.

Innerhalb des Antigen-bindenden Teils eines Antikörpers befinden sich komplementaritätsbestimmende Regionen (CDRs), die direkt mit dem Epitop des Antigens wechselwirken, und Gerüstregionen (FRs), die die Tertiärstruktur des Paratops aufrechterhalten. Sowohl in dem Fd-Fragment der schweren Kette als auch in der leichten Kette von IgG-Immunglobulinen befinden sich vier Gerilstregionen (FR1 bis FR4), die jeweils durch drei komplementaritätsbestimmende Regionen (CDR1 bis CDR3) getrennt sind. Die CDRs und insbesondere die CDR3-Regionen und noch mehr die CDR3-Region der schweren Kette sind größtenteils für die Antikörper-Spezifität verantwortlich.

Man weiß, dass die Nicht-CDR-Regionen eines Säuger-Antikörpers durch ähnliche Regionen von Antikörpern mit der gleichen oder einer anderen Spezifität ersetzt werden können, wobei die Spezifität für das Epitop des ursprünglichen Antikörpers erhalten bleibt. Dies ermöglichte die Entwicklung sogenannter "humanisierter" Antikörper, bei denen nicht-menschliche CDRs kovalent mit menschlichen FR- und/oder Fc/pFc'-Regionen für die Herstellung eines funktionellen Antikörpers verbunden sind.

Zum Beispiel beschreibt die WO 92/04381 die Herstellung und Verwendung von humanisierten RSV-Antikörpern aus Maus, bei denen mindestens ein Teil der FR-Regionen aus Maus durch FR-Regionen eines menschlichen Ursprungs ersetzt wurden. Solche Antikörper, einschließlich Fragmente intakter Antikörper mit einer Antigen-Bindefähigkeit werden oft als "chimäre" Antikörper bezeichnet.

Erfindungsgemäß werden auch F(ab')₂-, Fab-, Fv- und Fd-Fragmente von Antikörpern, chimäre Antikörper, bei denen die Fc- und/oder FR- und/oder CDR1- und/oder CDR2- und/oder leichte Kette-CDR3-Regionen durch homologe menschliche oder nicht-menschliche Sequenzen ersetzt wurden, chimäre F(ab')₂-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, chimäre Fab-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, und chimäre Fd-Fragment-Antikörper, bei denen die FR- und/oder CDR1- und/oder CDR2-Regionen durch homologe menschliche oder nicht-menschliche Sequenzen ersetzt wurden, dargestellt. Erfindungsgemäß sind auch sogenannte einzelkettige Antikörper denkbar.

Denkbar sind auch Polypeptide, die spezifisch an Tumor-assoziierte Antigene binden. Beispielsweise können solche Polypeptid-Bindestoffe durch degenerierte Peptid-Bibliotheken bereitgestellt werden, die einfach in Lösung in einer immobilisierten Form oder als Phagen-Display-Bibliotheken hergestellt werden können. Kombinatorische Bibliotheken aus Peptiden mit einer oder mehreren Aminosäuren können ebenfalls hergestellt werden. Ferner können Bibliotheken aus Peptoiden und nicht-peptidischen synthetischen Resten hergestellt werden.

Phagen-Display kann besonders wirksam bei der Identifizierung erfindungsgemäßer Bindepeptide sein. Dabei wird beispielsweise eine Phagen-Bibliothek (durch Verwendung beispielsweise des m13-, fd- oder lambda-Phagen) hergestellt, die Inserts einer Länge von 4 bis etwa 80 Aminosäureresten präsentiert. Es werden sodann Phagen ausgewählt, die Inserts tragen, die an das Tumor-assoziierte Antigen binden. Dieser Prozess kann über mehrere Zyklen einer Rückselektion von Phagen wiederholt werden, die an das Tumor-assoziierte Antigen binden. Wiederholte Runden führen zu einer Anreicherung von Phagen, die bestimmte Sequenzen tragen. Es kann eine Analyse von DNA-Sequenzen erfolgen, um die Sequenzen der exprimierten Polypeptide zu identifizieren. Der kleinste lineare Anteil der Sequenz, der an das Tumor-assoziierte Antigen bindet, kann bestimmt werden. Das "twohybrid-System" aus Hefe kann auch für die Identifizierung von Polypeptiden eingesetzt werden, die an ein Tumor-assoziiertes Antigen binden. Erfindungsgemäß beschriebene Tumor-assoziierte Antigene oder Fragmente davon können für ein Screening von Peptid-Bibliotheken, einschließlich Phagen-Display-Bibliotheken, eingesetzt werden, um Peptid-Bindepartner der Tumor-assoziierten Antigene zu identifizieren und selektieren. Solche Moleküle können beispielsweise für Screening-Assays, Aufreinigungsprotokolle, für eine Interferenz mit der Funktion des Tumor-assoziierten Antigens und für andere Zwecke, die dem Fachmann bekannt sind, verwendet werden.

Die vorstehend beschriebenen Antikörper und andere Bindemoleküle können beispielsweise für die Identifizierung von Gewebe verwendet werden, das ein Tumor-assoziiertes Antigen exprimiert. Antikörper können auch an spezifische diagnostische Stoffe für eine Darstellung von Zellen und Geweben gekoppelt werden, die Tumor-assoziierte Antigene exprimieren. Sie können ferner an therapeutisch nützliche Stoffe gekoppelt werden. Diagnostische Stoffe umfassen in nicht begrenzender Weise Bariumsulfat, Iocetaminsäure, Iopansäure, Calcium-Ipodat, Natrium-Diatrizoat, Meglumin-Diatrizoat, Metrizamid, Natrium-Tyropanoat und Radiodiagnostika, einschließlich Positronen-Emitter wie Fluorin-18 und Carbon-11, gamma-Emitter wie Iodin-123, Technitium-99m, Iod-131 und Indium-I11, Nuklide für magnetische Kernresonanz wie Fluorin und Gadolinium. Der Begriff "therapeutisch nützlicher Stoff" meint erfindungsgemäß jedes therapeutische Molekül, das wunschgemäß selektiv zu einer Zelle geführt wird, die ein oder mehrere Tumor-assoziierte Antigene exprimiert, einschließlich Antikrebsmittel, mit radioaktivem Iod versehene Verbindungen, Toxine, cytostatische oder cytolytische Arzneistoffe, usw. Antikrebsmittel umfassen beispielsweise Aminoglutethimid, Azathioprin, Bleomycinsulfat, Busulfan, Carmustin, Chlorambucil, Cisplatin, Cyclophosphamid, Cyclosporin, Cytarabidin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat. Weitere Antikrebsmittel sind beispielsweise in Goodman und Gilman, "The Pharmacological Basis of Therapeutics", 8. Auflage, 1990, McGraw-Hill, Inc., insbesondere Kapitel 52 (Antineoplastic Agents (Paul Calabresi und Bruce A. Chabner) beschrieben. Toxine können Proteine wie Pokeweed-antivirales Protein, Choleratoxin, Pertussistoxin, Ricin, Gelonin, Abrin, Diphtherie-Exotoxin oder *Pseudomonas*-Exotoxin sein. Toxinreste können auch Hochenergie-emittierende Radionuklide wie Kobalt-60 sein.

Der Begriff "Patient" bedeutet erfindungsgemäß Mensch, nicht menschlicher Primat oder ein anderes Tier, insbesondere Säugetier wie Kuh, Pferd, Schwein, Schaf, Ziege, Hund, Katze oder Nagetier wie Maus und Ratte. In einer besonders bevorzugten Ausführungsform ist der Patient ein Mensch.

Der Begriff "Erkrankung" betrifft erfindungsgemäß jeden pathologischen Zustand, bei dem Tumor-assoziierte Antigene exprimiert werden. "Abnormale Expression" bedeutet, dass die Expression gegenüber dem Zustand bei einem gesunden Individuum verändert, vorzugsweise erhöht ist. Eine Erhöhung der Expression betrifft eine Erhöhung um mindestens 10%, insbesondere mindestens 20%, mindestens 50% oder mindestens 100%. Denkbar ist, dass das Tumor-assoziierte Antigen nur in Gewebe eines erkrankten Individuums exprimiert, während die Expression bei einem gesunden Individuum reprimiert ist. Ein Beispiel einer solchen Erkrankung ist Krebs, insbesondere Seminome, Melanome, Teratome, Gliome, Kolorektal-, Brust-, Prostata-, Gebärmutter-, Ovarial-, und Lungenkrebs.

Eine biologische Probe kann erfindungsgemäß eine Gewebe- und/oder zelluläre Probe sein und kann für eine Verwendung in den verschiedenen, hier beschriebenen Verfahren in herkömmlicher Weise gewonnen werden, wie durch Gewebebiopsie, einschließlich Stanzbiopsie, und Entnahme von Blut, Bronchialaspirat, Urin, Fäces oder anderen Körperflüssigkeiten.

Der Begriff "immunreaktive Zelle" bedeutet erfindungsgemäß eine Zelle, die in eine Immunzelle (wie B-Zelle, T-Helferzelle oder cytolytische T-Zelle) bei geeigneter Stimulierung reifen kann. Immunreaktive Zellen umfassen CD34⁺ hämatopoietische Stammzellen, unreife und reife T-Zellen sowie unreife und reife B-Zellen. Falls die Herstellung cytolytischer oder Helfer T-Zellen, die ein Tumor-assoziiertes Antigen erkennen, gewünscht ist, wird die immunreaktive Zelle mit einer Zelle, die ein Tumor-assoziiertes Antigen exprimiert, unter Bedingungen in Kontakt gebracht, die eine Produktion, Differenzierung und/oder Selektion von cytolytischen sowie Helfer T-Zellen begünstigen. Die Differenzierung von T-Zell-Vorläufern in eine cytolytische T-Zelle bei einer Exposition gegenüber einem Antigen ist ähnlich zur klonalen Selektion des Immunsystems.

Die erfindungsgemäßen pharmazeutischen Zusammensetzungen werden im Allgemeinen in pharmazeutisch verträglichen Mengen und in pharmazeutisch verträglichen Zusammensetzungen verabreicht. Der Begriff "pharmazeutisch verträglich" betrifft ein nichttoxisches Material, das nicht mit der Wirkung des aktiven Bestandteils der pharmazeutischen Zusammensetzung wechselwirkt. Solche Zubereitungen können gewöhnlich Salze, Pufferstoffe, Konservierungsstoffe, Träger und gegebenenfalls andere therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind erfindungsgemäß umfasst. Solche pharmakologisch und pharmazeutisch verträglichen Salze umfassen in nicht begrenzender Weise diejenigen, die aus den nachstehenden Säuren hergestellt werden: Chlorwasserstoff-, Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Alkalimetall- oder Erdalkalimetallsalze wie Natrium-, Kalium- oder Calciumsalze hergestellt werden.

Eine erfindungsgemäße pharmazeutische Zusammensetzung kann einen pharmazeutisch verträglichen Träger umfassen. Der Begriff "pharmazeutisch verträglicher Träger" betrifft erfindungsgemäß einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der erfindungsgemäßen pharmazeutischen Zusammensetzung sind gewöhnlich derart, dass keine Interaktion auftritt, die die gewünschte pharmazeutische Wirksamkeit wesentlich beeinträchtigt.

Die erfindungsgemäßen pharmazeutischen Zusammensetzungen können geeignete Pufferstoffe wie Essigsäure in einem Salz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Erfindungsgemäße pharmazeutische Zusammensetzungen können beispielsweise in Form von Kapseln, Tabletten, Lutschpastillen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung des Wirkstoffs, die vorzugsweise mit dem Blut des Empfängers isotonisch ist. Verträgliche Träger und Lösungsmittel sind beispielsweise Ringer-Lösung und isotonische Natriumchloridlösung. Zusätzlich werden gewöhnlich sterile, fixierte Öle als Lösungs- oder Suspensionsmedium eingesetzt.

Die vorliegende Erfindung wird durch die nachstehenden Abbildungen und Beispiele ausführlich beschrieben, die ausschließlich der Erläuterung dienen und nicht begrenzend zu verstehen sind. Dem Fachmann sind aufgrund der Beschreibung und der Beispiele weitere Ausführungsformen zugänglich.

### Abbildungen:

**Abb. 1****: Schematische Darstellung der Klonierung von eCT.** Die Strategie umfasst die Identifizierung von Kandidaten-Genen (GOI = "Genes of interest") in Datenbanken und das Testen dieser Gene durch RT-PCR.
**Abb. 2****: Exon**-**Zusammensetzung von TPTE-Varianten.** Erfindungsgemäß wurden Splicevarianten identifiziert (SEQ ID NO:20, SEQ ID NO:21, SEQ ID NO:54, SEQ ID NO:55, SEQ ID NO:56, SEQ ID NO:57), die in testikulärem Gewebe und Tumoren exprimiert werden und Leserasterverschiebungen und somit veränderte Sequenzbereiche aufweisen.
**Abb. 3****: Alignment der möglichen TPTE-Proteine.** Alternative Splicing-Ereignisse resultieren in Veränderungen des kodierten Proteins, wobei das Leseraster grundsätzlich erhalten bleibt. Die putativen transmembrandomänen sind fettgedruckt, die katalytische Domäne ist eingerahmt.

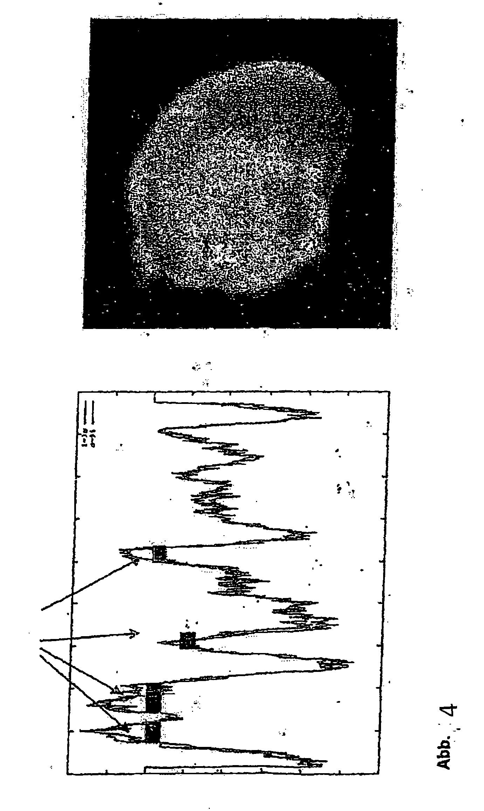

**Abb 4: Prädizierte Topologie von TPTE und subzelluläre Lokalisation an der Zelloberfläche von MCF-7 Zellen**
   Das linke Schema zeigt die 4 putativen Trabsmembran-Domänen (Pfeile) von TPTE. MCF-7 Zellen wurden transient mit einem TPTE-Expressions-Plasmid transfiziert. Das Antigen wurde mit TPTE-spezifischen Antikörpern detektiert und zeigte eine deutliche Kolokalisation mit an der Zelloberfläche lokalisierten MHC I Molekülen.

### Beispiele:

### Material und Methoden

Die Begriffe "*in silico*", "elektronisch" und "virtuell klonieren" beziehen sich rein auf die Nutzung von auf Datenbanken beruhenden Verfahren, mit denen auch Labor-experimentelle Vorgänge simuliert werden können.

Alle anderen Begriffe und Termini sind, falls nicht explizit anders definiert, so verwendet, wie sie der Fachmann versteht. Die genannten Techniken und Methoden erfolgen in an sich bekannter Weise und sind z.B. in Sambrook et al., Molecular Cloning: A Laboratory Manual, 2. Auflage (1989) Cold Spring Harbor Laboratory Press, Cold Spring Harbor, N. Y beschrieben. Alle Verfahren, die die Verwendung von Kits und Reagenzien einschließen, sind entsprechend den Angaben der Hersteller durchgeführt.

### Datamining-basierte Strategie zur Ermittlung von eCT (elektronisch klonierte Cancer/Testis-Gene)

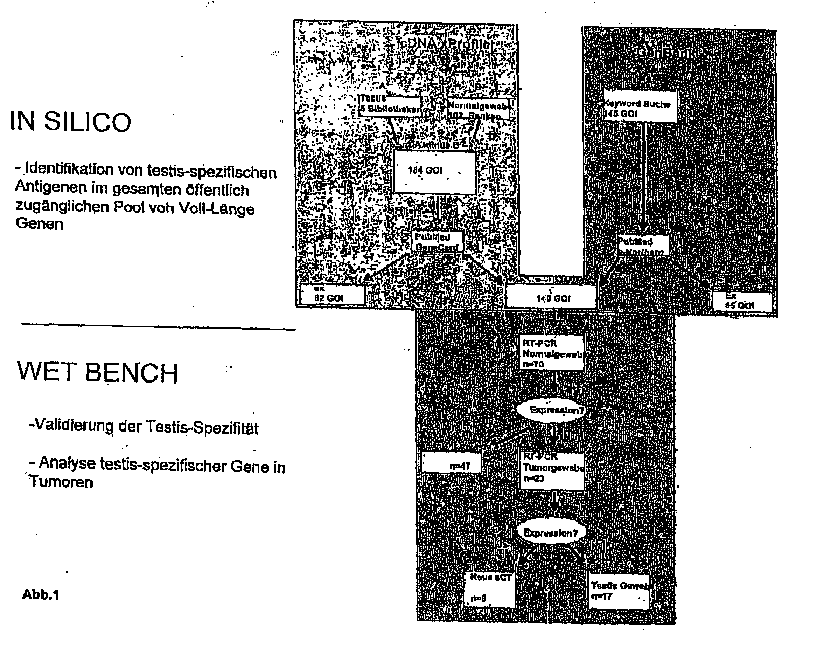

Zwei *in silico* Strategien nämlich GenBank-Schlagwort-Suche und der cDNAxProfiler wurden kombiniert (Abb. 1). Es wurde unter Nutzung des ENTREZ Search and Retrieval Systems des NCBI (http://www.ncbi.nlm.nih.gov/Entrez) eine Suche nach Kandidaten-Genen in der GenBank durchgeführt, die annotiert sind als spezifisch exprimiert in testikulärem Gewebe (Wheeler et al., Nucleic Acids Research 28:10-14, 2000).

Durch Suchabfragen mit den Schlagworten "testis-specific gene", "sperm-specific gene", "spermatogonia-specific gene" wurden Kandidatengene (GOI, genes of interest) aus den Datenbanken herausextrahiert. Die Suche wurde auf einen Teil der Gesamtinformation dieser Datenbanken eingeschränkt, indem als Limits "homo sapiens" für den Organismus und "mRNA" für die Molekülart eingesetzt wurden.

Die Liste der gefundenen GOI wurde kuratiert, indem unterschiedliche Bezeichnungen für dieselbe Sequenz ermittelt und solche Redundanzen behoben wurden.

Alle Kandidatengene, die sich durch die Schlagwort-Suche ergaben, wurden wiederum durch das Verfahren des "electronic Northern" (eNorthem) bezüglich ihrer Gewebeverteilung untersucht. Der eNorthern basiert darauf, dass die Sequenz eines GOI gegenüber einer EST-(expressed sequence tag) Datenbank (Adams et al., Science 252:1651, 1991) abgeglichen wird (http://www.ncbi.nlm.nih.gov/BLAST). Zu jedem EST, das sich als homolog zum eingegebenen GOI ergibt, lässt sich die Gewebeherkunft ermitteln und durch die Summe aller ESTs auf diese Weise eine vorläufige Einschätzung der Gewebeverteilung des GOI erreichen. Nur diejenigen GOI wurden weiteren Untersuchungen zugeführt, die keine Homologien zu EST aus nicht-testikulären Normalgeweben mit Ausnahme von Plazenta und fetalem Gewebe hatten. Für dies Beurteilung wurde auch berücksichtigt, dass es falsch-annotierte cDNA Banken in der öffentlichen Domäne gibt (Scheurle et al., Cancer Res. 60:4037-4043, 2000) (www.fau.edu/cmbb/publications/cancergenes6.htm).

Als zweites Datamining-Verfahren wurde der **cDNA xProfiler** des Cancer Genome Anatomy Projekts des NCBI (http://cgap.nci.nih.gov/Tissues/xProfiler) genutzt (Hillier et al., Genome Research 6:807-828, 1996; Pennisi, Science 276:1023-1024, 1997). Dieser erlaubt Pools von in Datenbanken abgelegten Transkriptomen durch logische Operatoren in Beziehung zueinander zu setzen. Wir haben einen Pool A definiert, dem alle aus Testis hergestellten Expressionsbibliotheken, unter Ausschluss von gemischten Bibliotheken zugeordnet wurden. Dem Pool B wurden alle cDNA-Bibliotheken zugeordnet, die von Normalgeweben mit Ausnahme von Testis, Ovar und Fetalgewebe hergestellt waren. Generell wurden alle cDNA-Banken unabhängig vom zugrundeliegenden Herstellungsverfahren genutzt, allerdings lediglich solche mit einer Mächtigkeit > 1000 zugelassen. Mittels des BUT NOT Operators wurde Pool B digital von Pool A subtrahiert. Auch das Set der auf diese Weise gefundenen GOI wurde eNorthem-Studien unterzogen, sowie durch eine Literaturrecherche abgesichert. Dieses kombinierte Datamining schließt alle etwa 13 000 Vollänge-Gene in der öffentlichen Domäne ein und prädiziert aus diesen insgesamt 140 Gene mit potentieller Testis-spezifischer Expression. Unter diesen waren 25 bereits bekannte Gene der CT-Genklasse, was die Effizienz unserer Strategie unterstreicht.

Alle anderen Gene wurden zunächst durch spezifische RT-PCR in Normalgeweben evaluiert. Alle GOI, die sich als in nicht-testikulären Normalgeweben exprimiert erwiesen, hatten als Falsch-Positive zu gelten und wurden aus weiteren Untersuchungen ausgeschlossen. Die verbliebenen wurden in einem großen Panel an verschiedensten Tumorgeweben untersucht. Die unten dargestellten Antigene erwiesen sich dabei als ektop in Tumorzellen aktiviert.

### RNA-Extraktion, Herstellung von poly-d(T) geprimter cDNA und RT-PCR Analyse

Gesamt-RNA aus nativem Gewebematerial wurde unter Verwendung von Guanidiumisothiocyanate als chaotrophem Agens extrahiert (Chomczynski & Sacchi, Anal. Biochem. 162:156-9, 1987). Nach Extraktion mit saurem Phenol und Fällung mit Isopropanol wurde die RNA in DEPC-behandeltem Wasser gelöst.

Aus 2-4 µg Gesamt-RNA wurde in einem 20µl Reaktionsansatz mittels Superscript II (Invitrogen) entsprechend den Angaben des Herstellers eine Erststrang-cDNA-Synthese durchgeführt. Als Primer wurde ein dT(18) Oligonukleotid verwendet. Integrität und Qualität der cDNA wurden durch Amplifikation von p53 in einer 30 Zyklen-PCR (sense CGTGAGCGCTTCGAGATGTTCCG, antisense CCTAACCAGCTGCCCAACTGTAG, Hybridisierungstemperatur 67°C) überprüft.

Es wurde ein Archiv aus Erststrang-cDNAs aus einer Reihe von Normalgeweben und Tumorentitäten hergestellt. Für Expressionsstudien wurden 0,5 µl dieser cDNAs in einem 30µl Reaktionsansatz mit GOI-spezifischen Primern (siehe unten) und 1 U HotStarTaq DNA Polymerase (Qiagen) amplifiziert. Der Reaktionsansatz enthielt jeweils 0,3 mM dNTPs, je 0,3 µM jeden Primers und 3 µl 10 x Reaktionspuffer.

Die Primer wurden so ausgewählt, dass sie in 2 verschiedenen Exons liegen und die Beseitigung der Interferenz durch kontaminierende genomische DNA als Grund für falsch positive Resultate wurde durch Testen von nicht revers transkribierter DNA als Matritze bestätigt. Nach 15 Minuten bei 95°C zur Aktivierung der HotStarTaq DNA Polymerase wurden 35 Zyklen PCR durchgeführt (1 min 94°C, 1 min jeweilige Hybridisierungstemperatur, 2 min 72°C und abschließende Elongation bei 72°C für 6 min). 20µl dieser Reaktion wurden auf einem mit Ethidiumbromid gefärbten Agarosegel aufgetrennt und analysiert.

Folgende Primer wurden für die Expressionsanalyse von TPTE bei der angegebenen Hybridisierunestemperatur verwendet.
TPTE (64°C)
sense TGGATGTCACTCTCATCCTTG, antisense CCATAGTTCCTGTTCTATCTG

### Klonierung und Sequenzanalyse

Klonierung von Vollängen bzw. Genfragmenten erfolgte nach gängigen Methoden. Zur Ermittlung der Sequenz wurden entsprechende Antigene mittels der Proofreading-Polymerase pfu (Stratagene) amplifiziert. Nach Beendigung der PCR wurde Adenosin mittels HotStarTaq DNA Polymerase an die Enden des Amplikons ligiert, um die Fragmente entsprechend den Angaben des Herstellers in den TOPO-TA Vektor zu klonieren. Die Sequenzierung wurde durch einen kommerziellen Service durchgeführt. Die Sequenzen wurden mittels gängiger Prädiktionsprogramme und Algorithmen analysiert.

### Beispiel 1: Identifizierung von TPTE als neues Tumorantigen

Die Sequenzen des Transkripts von TPTE (SEQ ID NO:19) und seines Translationsprodukt (SEQ ID NO:22) sind in GenBank unter der Zugangsnummer NM_013315 veröffentlicht (Walker, S.M. et al., Biochem. J. 360(Pt 2):277-83, 2001; Guipponi M. et al., Hum. Genet. 107(2):127-31, 2000; Chen H. et al., Hum. Genet. 105(5):399-409, 1999). TPTE wurde als Gen, das für eine mögliche transmembrane Tyrosinphosphatase kodiert, mit Testisspezifischer Expression beschrieben, die in der perizentromeren Region der Chromosomen 21, 13, 15, 22, and Y liegt (Chen, H. et al., Hum. Genet. 105:399-409, 1999). Erfindungsgemäße Abgleichuntersuchungen zeigen zusätzlich homologe genomische Sequenzen auf den Chromosomen 3 und 7.

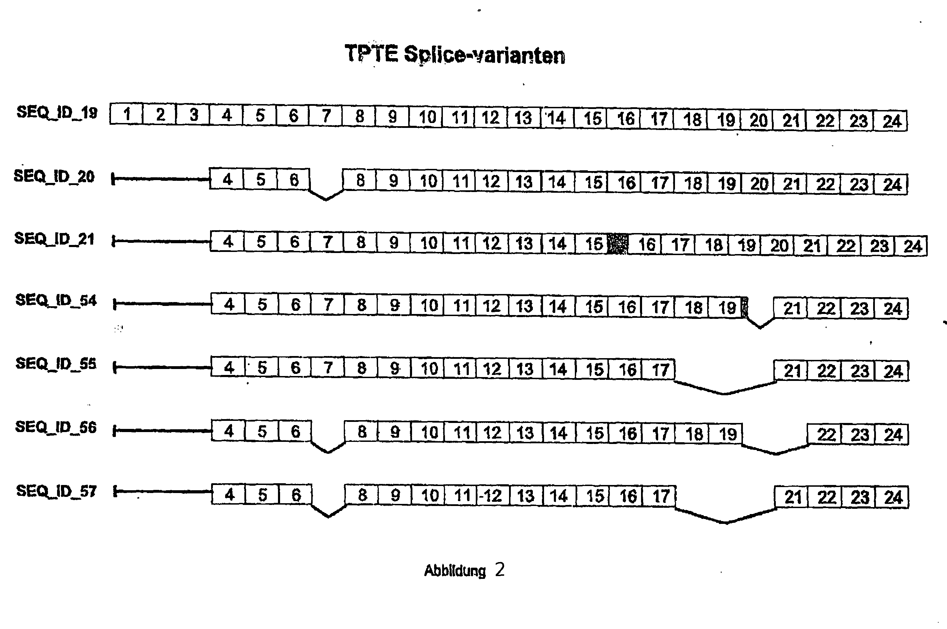

Basierend auf der Sequenz von TPTE (SEQ ID NO:19) wurden erfindungsgemäß PCR-Primer generiert (5'-TGGATGTCACTCTCATCCTTG-3' (SEQ ID NO: 27) and 5'-CCATAGTTCCTGTTCTATCTG_-3'- (SEQ ID NO: 281) und für RT-PCR-Analysen (95° 15min; 94° 1 min; 63° 1 min; 72° 1 min; 35 Zyklen) in einer Reihe von humanen Geweben eingesetzt. Es zeigte sich, dass die Expression in Normalgeweben auf Testis beschränkt ist. Wie für die anderen eCT beschrieben, konnte erfindungsgemäß gezeigt werden, dass TPTE-Varianten ektop in einer Reihe von Tumorgeweben aktiviert werden; vgl. Tabelle 1. Erfindungsgemäß wurden für TPTE weitere Splicevarianten identifiziert (SEQ ID NO:20, SEQ ID NO:21, SEQ ID NO:54, SEQ ID NO:55, SEQ ID NO:56, SEQ ID NO:57), die in testikulärem Gewebe und Tumoren exprimiert werden und Leserasterverschiebungen und somit veränderte Sequenzbereiche aufweisen (Abb. 2).

**Tabelle 2. Expression von TPTE in Tumoren**

| **Gewebe** | **insgesamt getestet** | **Positiv** | **%** |
|---|---|---|---|
| **Melanom** | **18** | **9** | **50** |
| **Mammakarzinome** | **20** | **4** | **20** |
| **Kolorektale Tumoren** | **20** | **0** | **0** |
| **Prostatakarzinome** | **8** | **3** | **38** |
| **Bronchialkarzinome** | **23** | **9** | **39** |
| **Nierenzellkarzinome** | **7** | **0** | **0** |
| **Ovarialkarzinome** | **7** | **2** | **29** |
| **Schilddrüsenkarzinome** | **4** | **0** | **0** |
| **Zervixkarzimome** | **6** | **1** | **17** |
| **Melanom Zellinien** | **8** | **4** | **50** |
| **Bronchialkarzinom Zellinien** | **6** | **2** | **33** |
| **Mammakarzinom Zellinien** | **5** | **4** | **80** |

Die genomische Sequenz von TPTE besteht aus 24 Exons (Zugangsnummer NT_02943 0). Das in SEQ ID NO:19 gezeigte Transkript enthält alle diese Exons. Die in SEQ ID NO:20 gezeigte Splicevariante entsteht durch heraussplicen von Exon 7. Die in SEQ ID NO:21 gezeigte Splicevariante zeigt eine partielle Inkorporation eines Introns, das auf Exon 15 folgt. Wie aus den Varianten SEQ ID NO:54, SEQ ID NO:55, SEQ ID NO:56, SEQ ID NO:57 ersichtlich, können alternativ auch Exons 18, 19, 20 und 21 herausgesplict werden.

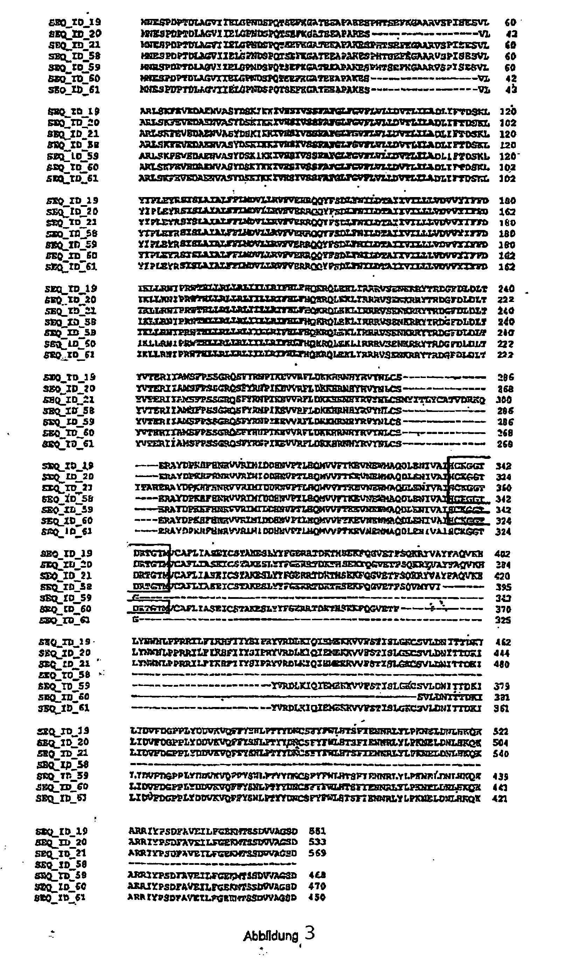

Diese alternativen Splicing-Ereignisse resultieren in Veränderungen des kodierten Proteins, wobei das Leseraster grundsätzlich erhalten bleibt (Abb. 3). Beispielsweise weist das Translationsprodukt, das von der in SEQ ID NO:20 gezeigten Sequenz kodiert wird (SEQ ID NO:23) eine Deletion von 13 Aminosäuren im Vergleich zu der in SEQ ID NO:22 gezeigten Sequenz auf. Das Translationsprodukt, das von der in SEQ ID NO:21 gezeigten Sequenz kodiert wird (SEQ ID NO:24) trägt eine zusätzliche Insertion im zentralen Bereich des Moleküls und unterscheidet sich dadurch um 14 Aminosäuren von den anderen Varianten. Die Translationsprodukte der Varianten SEQ ID NO:S4, SEQ ID NO:55, SEQ ID NO:56, SEQ ID NO:57 nämlich die Proteine SEQ ID NO:58, SEQ ID NO:59, SEQ ID NO:60, SEQ ID NO:61 sind ebenfalls verändert.

Analysen zur Prädiktion funktioneller Domänen ergeben für SEQ ID NO:22, SEQ ID NO:23, SEQ ID NO:24, SEQ ID NO:58, SEQ ID NO:60, aber nicht für SEQ ID NO:59, SEQ ID NO:61, das Vorhandensein einer Tyrosinphosphatase-Domäne. Für alle Varianten werden 3-4 Transmembrandomänen prädiziert (Abb. 3).

Die Analyse der TPTE-Antigen-Expression mit spezifischen Antikörpern bestätigte die selektive Expression in Testis sowie in einer Reihe von unterschiedlichen Tumoren. Durch Kolokalisationsuntersuchungen zeigte sich zudem, dass TPTE erfindungsgemäß mit Klasse I Immunglobulinen an der Zelloberfläche von Tumorzellen lokalisiert ist. Bisher war TPTE lediglich als Golgi-assoziiertes Protein beschrieben worden. Aufgrund der Expression von TPTE auf der Zelloberfläche von Tumorzellen eignet sich dieses Tumorantigen erfindungsgemäß als hervorragendes Target für die Entwicklung diagnostischer und therapeutischer monoklonaler Antikörper. Aufgrund der prädizierten Membrantopologie von TPTE eignen sich für diesen Zweck erfindungsgemäß insbesondere die extrazellulär exponierten Bereiche. Dies umfasst erfindungsgemäß die Peptide FTDSKLYIPLEYRS (SEQ ID NO:81) und FDIKLLRNIPRWT (SEQ ID NO:82). Darüber hinaus konnte gezeigt werden, dass TPTE die Migration von Tumorzellen fördert. Hierzu wurden Tumorzellen, die unter Kontrolle eines eukaryontischen Promotors mit TPTE transfiziert wurden, und Kontrollzellen in sog. Boyden Chamber Migrationsversuchen darauf untersucht, ob sie eine gerichtete Migration zeigen. TPTE transfizierte Zellen wiesen dabei in 4 unabhängigen Versuchen erfindungsgemäß eine deutlich (3-fach) gesteigerte Migration auf. Diese funktionellen Daten deuten darauf hin, dass TPTE bei der Metastasierung von Tumoren eine wesentliche Rolle spielt. Somit könnten Verfahren, die erfindungsgemäß die endogene Aktivität von TPTE in Tumorzellen inhibieren, z.B. durch den Einsatz von Antisene RNA, unterschiedlicher Methoden der RNA-Interferenz (RNAi) mittels Expressionsvektoren oder Retroviren, sowie durch den Einsatz von kleinen Moleküle, zu einer reduzierten Meatstasierung führen und somit therapeutisch sehr bedeutsam sein. Ein kausaler Zusammenhang zwischen der Aktivität einer Phosphatase in Tumoren und gesteigerter Migration und erhöhter Metastasebildung konnte kürzlich für die Tyrosinphosphatase PTEN etabliert werden (lijima und Devreotes Cell 109:599-610, 2002).

### SEQUENCE LISTING

<110> Sahin Dr., Ugur
   Türeci Dr., Özlem
   Koslowski Dr., Michael
<120> Differentell in Tumoren exprimierte Genprodukte und deren Verwendung
<130> 342-3PCT
<160> 100
<170> PatentIn version 3.1
<210> 19
   <211> 2168
   <212> DNA
   <213> Homo sapiens
<400> 19
   gaatccgcgg ggagggcaca acagctgcta cctgaacagt ttctgaccca acagttaccc     60
   agcgccggac tcgctgcgcc ccggcggctc tagggacccc cggcgcctac acttagctcc    120
   gcgcccgaga gaatgttgga ccgacgacac aagacctcag acttgtgtta ttctagcagc    180
   tgaacacacc ccaggctctt ctgaccggca gtggctctgg aagcagtctg gtgtatagag    240
   ttatggattc actaccagat tctactgtat gctcttgaca actatgacca caatggtcca    300
   cccacaaatg aattatcagg agtgaaccca gaggcacgta tgaatgaaag tcctgatccg    360
   actgacctgg cgggagtcat cattgagctc ggccccaatg acagtccaca gacaagtgaa    420
   tttaaaggag caaccgagga ggcacctgcg aaagaaagcc cacacacaag tgaatttaaa    480
   ggagcagccc gggtgtcacc tatcagtgaa agtgtgttag cacgactttc caagtttgaa    540
   gttgaagatg ctgaaaatgt tgcttcatat gacagcaaga ttaagaaaat tgtgcattca    600
   attgtatcat cctttgcatt tggactattt ggagtttttc tggtcttact ggatgtcact    660
   ctcatccttg ccgacctaat tttcactgac agcaaacttt atattccttt ggagtatcgt    720
   tctatttctc tagctattgc cttatttttt ctcatggatg ttcttcttcg agtatttgta    780
   gaaaggagac agcagtattt ttctgactta tttaacattt tagatactgc cattattgtg    840
   attcttctgc tggttgatgt cgtttacatt ttttttgaca ttaagttgct taggaatatt    900
   cccagatgga cacatttact tcgacttcta cgacttatta ttctgttaag aatttttcat    960
   ctgtttcatc aaaaaagaca acttgaaaag ctgataagaa ggcgggtttc agaaaacaaa   1020
   aggcgataca caagggatgg atttgaccta gacctcactt acgttacaga acgtattatt   1080
   gctatgtcat ttccatcttc tggaaggcag tctttctata gaaatccaat caaggaagtt   1140
   gtgcggtttc tagataagaa acaccgaaac cactatcgag tctacaatct atgcagtgaa   1200
   agagcttacg atcctaagca cttccataat agggtcgtta gaatcatgat tgatgatcat   1260
   aatgtcccca ctctacatca gatggtggtt ttcaccaagg aagtaaatga gtggatggct   1320
   caagatcttg aaaacatcgt agcgattcac tgtaaaggag gcacagatag aacaggaact   1380
   atggtttgtg ccttccttat tgcctctgaa atatgttcaa ctgcaaagga aagcctgtat   1440
   tattttggag aaaggcgaac agataaaacc cacagcgaaa aatttcaggg agtagaaact   1500
   ccttctcaga agagatatgt tgcatatttt gcacaagtga aacatctcta caactggaat   1560
   ctccctccaa gacggatact ctttataaaa cacttcatta tttattcgat tcctcgttat   1620
   gtacgtgatc taaaaatcca aatagaaatg gagaaaaagg ttgtcttttc cactatttca   1680
   ttaggaaaat gttcggtact tgataacatt acaacagaca aaatattaat tgatgtattc   1740
   gacggtccac ctctgtatga tgatgtgaaa gtgcagtttt tctattcgaa tcttcctaca   1800
   tactatgaca attgctcatt ttacttctgg ttgcacacat cttttattga aaataacagg   1860
   ctttatctac caaaaaatga attggataat ctacataaac aaaaagcacg gagaatttat   1920
   ccatcagatt ttgccgtgga gatacttttt ggcgagaaaa tgacttccag tgatgttgta   1980
   gctggatccg attaagtata gctccccctt ccccttctgg gaaagaatta tgttctttcc   2040
   aaccctgcca catgttcata tatcctaaat ctatcctaaa tgttcccttg aagtatttat   2100
   ttatgtttat atatgtttat acatgttctt caataaatct attacatata tataaaaaaa   2160
   aaaaaaaa                                                            2168
<210> 20
   <211> 2114
   <212> DNA
   <213> Homo sapiens
<400> 20
   gaatccgcgg ggagggcaca acagctgcta cctgaacagt ttctgaccca acagttaccc     60
   agcgccggac tcgctgcgcc ccggcggctc tagggacccc cggcgcctac acttagctcc    120
   gcgcccgaga gaatgttgga ccgacgacac aagacctcag acttgtgtta ttctagcagc    180
   tgaacacacc ccaggctctt ctgaccggca gtggctctgg aagcagtctg gtgtatagag    240
   ttatggattc actaccagat tctactgtat gctcttgaca actatgacca caatggtcca    300
   cccacaaatg aattatcagg agtgaaccca gaggcacgta tgaatgaaag tcctgatccg    360
   actgacctgg cgggagtcat cattgagctc ggccccaatg acagtccaca gacaagtgaa    420
   tttaaaggag caaccgagga ggcacctgcg aaagaaagtg tgttagcacg actttccaag    480
   tttgaagttg aagatgctga aaatgttgct tcatatgaca gcaagattaa gaaaattgtg    540
   cattcaattg tatcatcctt tgcatttgga ctatttggag ttttcctggt cttactggat    600
   gtcactctca tccttgccga cctaattttc actgacagca aactttatat tcctttggag    660
   tatcgttcta tttctctagc tattgcctta ttttttctca tggatgttct tcttcgagta    720
   tttgtagaaa ggagacagca gtatttttct gacttattta acattttaga tactgccatt    780
   attgtgattc ttctgctggt tgatgtcgtt tacatttttt ttgacattaa gttgcttagg    840
   aatattccca gatggacaca tttacttcga cttctacgac ttattattct gttaagaatt    900
   tttcatctgt ttcatcaaaa aagacaactt gaaaagctga taagaaggcg ggtttcagaa 960
   aacaaaaggc gatacacaag ggatggattt gacctagacc tcacttacgt tacagaacgt 1020
   attattgcta tgtcatttcc atcttctgga aggcagtctt tctatagaaa tccaatcaag 1080
   gaagttgtgc ggtttctaga taagaaacac cgaaaccact atcgagtcta caatctatgc 1140
   agtgaaagag cttacgatcc taagcacttc cataataggg tcgttagaat catgattgat 1200
   gatcataatg tccccactct acatcagatg gtggttttca ccaaggaagt aaatgagtgg 1260
   atggctcaag atcttgaaaa catcgtagcg attcactgta aaggaggcac agatagaaca 1320
   ggaactatgg tttgtgcctt ccttattgcc tctgaaatat gttcaactgc aaaggaaagc 1380
   ctgtattatt ttggagaaag gcgaacagat aaaacccaca gcgaaaaatt tcagggagta 1440
   gaaactcctt ctcagaagag atatgttgca tattttgcac aagtgaaaca tctctacaac 1500
   tggaatctcc ctccaagacg gatactcttt ataaaacact tcattattta ttcgattcct 1560
   cgttatgtac gtgatctaaa aatccaaata gaaatggaga aaaaggttgt cttttccact 1620
   atttcattag gaaaatgttc ggtacttgat aacattacaa cagacaaaat attaattgat 1680
   gtattcgacg gtccacctct gtatgatgat gtgaaagtgc agtttttcta ttcgaatctt 1740
   cctacatact atgacaattg ctcattttac ttctggttgc acacatcttt tattgaaaat 1800
   aacaggcttt atctaccaaa aaatgaattg gataatctac ataaacaaaa agcacggaga 1860
   atttatccat cagattttgc cgtggagata ctttttggcg agaaaatgac ttccagtgat 1920
   gttgtagctg gatccgatta agtatagctc ccccttcccc ttctgggaaa gaattatgtt 1980
   ctttccaacc ctgccacatg ttcatatatc ctaaatctat cctaaatgtt cccttgaagt 2040
   atttatttat gtttatatat gtttatacat gttcttcaat aaatctatta catatatata 2100
   aaaaaaaaaa aaaa 2114
<210> 21
   <211> 2222
   <212> DA
   <213> Homo sapiens
<400> 21
   gaatccgcgg ggagggcaca acagctgcta cctgaacagt ttctgaccca acagttaccc 60
   agcgccggac tcgctgcgcc ccggcggctc tagggacccc cggcgcctac acttagctcc 120
   gcgcccgaga gaatgttgga ccgacgacac aagacctcag acttgtgtta ttctagcagc 180
   tgaacacacc ccaggctctt ctgaccggca gtggctctgg aagcagtctg gtgtatagag 240
   ttatggattc actaccagat tctactgtat gctcttgaca actatgacca caatggtcca 300
   cccacaaatg aattatcagg agtgaaccca gaggcacgta tgaatgaaag tcctgatccg 360
   actgacctgg cgggagtcat cattgagctc ggccccaatg acagtccaca gacaagtgaa 420
   tttaaaggag caaccgagga ggcacctgcg aaagaaagcc cacacacaag tgaatttaaa    480
   ggagcagccc gggtgtcacc tatcagtgaa agtgtgttag cacgactttc caagtttgaa    540
   gttgaagatg ctgaaaatgt tgcttcatat gacagcaaga ttaagaaaat tgtgcattca    600
   attgtatcat cctttgcatt tggactattt ggagttttcc tggtcttact ggatgtcact    660
   ctcatccttg ccgacctaat tttcactgac agcaaacttt atattccttt ggagtatcgt    720
   tctatttctc tagctattgc cttatttttt ctcatggatg ttcttcttcg agtatttgta    780
   gaaaggagac agcagtattt ttctgactta tttaacattt tagatactgc cattattgtg    840
   attcttctgc tggttgatgt cgtttacatt ttttttgaca ttaagttgct taggaatatt    900
   cccagatgga cacatttact tcgacttcta cgacttatta ttctgttaag aatttttcat    960
   ctgtttcatc aaaaaagaca acttgaaaag ctgataagaa ggcgggtttc agaaaacaaa   1020
   aggcgataca caagggatgg atttgaccta gacctcactt acgttacaga acgtattatt   1080
   gctatgtcat ttccatcttc tggaaggcag tctttctata gaaatccaat caaggaagtt   1140
   gtgcggtttc tagataagaa acaccgaaac cactatcgag tctacaatct atgcagtatg   1200
   tacattactc tatattgtgc tactgtagat agaaaacaga ttactgcacg tgaaagagct   1260
   tacgatccta agcacttcca taatagggtc gttagaatca tgattgatga tcataatgtc   1320
   cccactctac atcagatggt ggttttcacc aaggaagtaa atgagtggat ggctcaagat   1380
   cttgaaaaca tcgtagcgat tcactgtaaa ggaggcacag atagaacagg aactatggtt   1440
   tgtgccttcc ttattgcctc tgaaatatgt tcaactgcaa aggaaagcct gtattatttt   1500
   ggagaaaggc gaacagataa aacccacagc gaaaaatttc agggagtaga aactccttct   1560
   cagaagagat atgttgcata ttttgcacaa gtgaaacatc tctacaactg gaatctccct   1620
   ccaagacgga tactctttat aaaacacttc attatttatt cgattcctcg ttatgtacgt   1680
   gatctaaaaa tccaaataga aatggagaaa aaggttgtct tttccactat ttcattagga   1740
   aaatgttcgg tacttgataa cattacaaca gacaaaatat taattgatgt attcgacggt   1800
   ccacctctgt atgatgatgt gaaagtgcag tttttctatt cgaatcttcc tacatactat   1860
   gacaattgct cattttactt ctggttgcac acatctttta ttgaaaataa caggctttat   1920
   ctaccaaaaa atgaattgga taatctacat aaacaaaaag cacggagaat ttatccatca   1980
   gattttgccg tggagatact ttttggcgag aaaatgactt ccagtgatgt tgtagctgga    2040
   tccgattaag tatagctccc ccttcccctt ctgggaaaga attatgttct ttccaaccct   2100
   gccacatgtt catatatcct aaatctatcc taaatgttcc cttgaagtat ttatttatgt   2160
   ttatatatgt ttatacatgt tcttcaataa atctattaca tatatataaa aaaaaaaaaa   2220
   aa                                                                  2222
<210> 22
   <211> 551
   <212> PRT
   <213> Homo sapiens
<400> 22
   Met Asn Glu Ser Pro Asp Pro Thr Asp Leu Ala Gly Val Ile Ile Glu
   1               5                   10                  15
   Leu Gly Pro Asn Asp Ser Pro Gln Thr Ser Glu Phe Lys Gly Ala Thr
               20                  25                  30
   Glu Glu Ala Pro Ala Lys Glu Ser Pro His Thr Ser Glu Phe Lys Gly
           35                  40                  45
   Ala Ala Arg Val Ser Pro Ile Ser Glu Ser Val Leu Ala Arg Leu Ser
       50                  55                  60
   Lys Phe Glu Val Glu Asp Ala Glu Asn Val Ala Ser Tyr Asp Ser Lys
   65                  70                  75                  80
   Ile Lys Lys Ile Val His Ser Ile Val Ser Ser Phe Ala Phe Gly Leu
                   85                  90                  95
   Phe Gly Val Phe Leu Val Leu Leu Asp Val Thr Leu Ile Leu Ala Asp
               100                 105                 110
   Leu Ile Phe Thr Asp Ser Lys Leu Tyr Ile Pro Leu Glu Tyr Arg Ser
           115                 120                 125
   Ile Ser Leu Ala Ile Ala Leu Phe Phe Leu Met Asp Val Leu Leu Arg
       130                 135                 140
   Val Phe Val Glu Arg Arg Gln Gln Tyr Phe Ser Asp Leu Phe Asn Ile
   145                 150                 155                 160
   Leu Asp Thr Ala Ile Ile Val Ile Leu Leu Leu Val Asp Val Val Tyr
                   165                 170                 175
   Ile Phe Phe Asp Ile Lys Leu Leu Arg Asn Ile Pro Arg Trp Thr His
               180                 185                 190
   Leu Leu Arg Leu Leu Arg Leu Ile Ile Leu Leu Arg Ile Phe His Leu
           195                 200                 205
   Phe His Gln Lys Arg Gln Leu Glu Lys Leu Ile Arg Arg Arg Val Ser
       210                 215                 220
   Glu Asn Lys Arg Arg Tyr Thr Arg Asp Gly Phe Asp Leu Asp Leu Thr
   225                 230                 235                 240
   Tyr Val Thr Glu Arg Ile Ile Ala Met Ser Phe Pro Ser Ser Gly Arg
                   245                 250                 255
   Gln Ser Phe Tyr Arg Asn Pro Ile Lys Glu Val Val Arg Phe Leu Asp
               260                 265                 270
   Lys Lys His Arg Asn His Tyr Arg Val Tyr Asn Leu Cys Ser Glu Arg
           275                 280                 285
   Ala Tyr Asp Pro Lys His Phe His Asn Arg Val Val Arg Ile Met Ile
       290                 295                 300
   Asp Asp His Asn Val Pro Thr Leu His Gln Met Val Val Phe Thr Lys
   305                 310                 315                 320
   Glu Val Asn Glu Trp Met Ala Gln Asp Leu Glu Asn Ile Val Ala Ile
                   325                 330                 335
   His Cys Lys Gly Gly Thr Asp Arg Thr Gly Thr Met Val Cys Ala Phe
               340                 345                 350
   Leu Ile Ala Ser Glu Ile Cys Ser Thr Ala Lys Glu Ser Leu Tyr Tyr
           355                 360                 365
   Phe Gly Glu Arg Arg Thr Asp Lys Thr His Ser Glu Lys Phe Gln Gly
       370                 375                 380
   Val Glu Thr Pro Ser Gln Lys Arg Tyr Val Ala Tyr Phe Ala Gln Val
   385                 390                 395                 400
   Lys His Leu Tyr Asn Trp Asn Leu Pro Pro Arg Arg Ile Leu Phe Ile
                   405                 410                 415
   Lys His Phe Ile Ile Tyr Ser Ile Pro Arg Tyr Val Arg Asp Leu Lys
               420                 425                 430
   Ile Gln Ile Glu Met Glu Lys Lys Val Val Phe Ser Thr Ile Ser Leu
           435                 440                 445
   Gly Lys Cys Ser Val Leu Asp Asn Ile Thr Thr Asp Lys Ile Leu Ile
       450                 455                 460
   Asp Val Phe Asp Gly Pro Pro Leu Tyr Asp Asp Val Lys Val Gln Phe
   465                 470                 475                 480
   Phe Tyr Ser Asn Leu Pro Thr Tyr Tyr Asp Asn Cys Ser Phe Tyr Phe
                   485                 490                 495
   Trp Leu His Thr Ser Phe Ile Glu Asn Asn Arg Leu Tyr Leu Pro Lys
               500                 505                 510
   Asn Glu Leu Asp Asn Leu His Lys Gln Lys Ala Arg Arg Ile Tyr Pro
   515 520 525
   Ser Asp Phe Ala Val Glu Ile Leu Phe Gly Glu Lys Met Thr Ser Ser
   530 535 540
   Asp Val Val Ala Gly Ser Asp
   545 550
<210> 23
   <211> 533
   <212> PRT
   <213> Homo sapiens
<400> 23
<210> 24
   <211> 569
   <212> PRT
   <213> Homo sapiens
<400> 24
   Met Asn Glu Ser Pro Asp Pro Thr Asp Leu Ala Gly Val Ile Ile Glu
   1 5 10 15
   Leu Gly Pro Asn Asp Ser Pro Gln Thr Ser Glu Phe Lys Gly Ala Thr
   20 25 30
   Glu Glu Ala Pro Ala Lys Glu Ser Pro His Thr Ser Glu Phe Lys Gly
   35 40 45
   Ala Ala Arg Val Ser Pro Ile Ser Glu Ser Val Leu Ala Arg Leu Ser
   50 55 60
   Lys Phe Glu Val Glu Asp Ala Glu Asn Val Ala Ser Tyr Asp Ser Lys
   65 70 75 80
   Ile Lys Lys Ile Val His Ser Ile Val Ser Ser Phe Ala Phe Gly Leu
   85 90 95
   Phe Gly Val Phe Leu Val Leu Leu Asp Val Thr Leu Ile Leu Ala Asp
   100 105 110
   Leu Ile Phe Thr Asp Ser Lys Leu Tyr Ile Pro Leu Glu Tyr Arg Ser
   115 120 125
   Ile Ser Leu Ala Ile Ala Leu Phe Phe Leu Met Asp Val Leu Leu Arg
   130 135 140
   Val Phe Val Glu Arg Arg Gln Gln Tyr Phe Ser Asp Leu Phe Asn Ile
   145 150 155 160
   Leu Asp Thr Ala Ile Ile Val Ile Leu Leu Leu Val Asp Val Val Tyr
   165 170 175
   Ile Phe Phe Asp Ile Lys Leu Leu Arg Asn Ile Pro Arg Trp Thr His
   180 185 190
   Leu Leu Arg Leu Leu Arg Leu Ile Ile Leu Leu Arg Ile Phe His Leu
   195 200 205
   Phe His Gln Lys Arg Gln Leu Glu Lys Leu Ile Arg Arg Arg Val Ser
   210 215 220
   Glu Asn Lys Arg Arg Tyr Thr Arg Asp Gly Phe Asp Leu Asp Leu Thr
   225 230 235 240
   Tyr Val Thr Glu Arg Ile Ile Ala Met Ser Phe Pro Ser Ser Gly Arg
   245 250 255
   Gln Ser Phe Tyr Arg Asn Pro Ile Lys Glu Val Val Arg Phe Leu Asp
   260 265 270
   Lys Lys His Arg Asn His Tyr Arg Val Tyr Asn Leu Cys Ser Met Tyr
   275 280 285
   Ile Thr Leu Tyr Cys Ala Thr Val Asp Arg Lys Gln Ile Thr Ala Arg
   290 295 300
   Glu Arg Ala Tyr Asp Pro Lys His Phe His Asn Arg Val Val Arg Ile
   305 310 315 320
   Met Ile Asp Asp His Asn Val Pro Thr Leu His Gln Met Val Val Phe
   325 330 335
   Thr Lys Glu Val Asn Glu Trp Met Ala Gln Asp Leu Glu Asn Ile Val
   340 345 350
   Ala Ile His Cys Lys Gly Gly Thr Asp Arg Thr Gly Thr Met Val Cys
   355 360 365
   Ala Phe Leu Ile Ala Ser Glu Ile Cys Ser Thr Ala Lys Glu Ser Leu
   370 375 380
   Tyr Tyr Phe Gly Glu Arg Arg Thr Asp Lys Thr His Ser Glu Lys Phe
   385 390 395 400
   Gln Gly Val Glu Thr Pro Ser Gln Lys Arg Tyr Val Ala Tyr Phe Ala
   405 410 415
   Gln Val Lys His Leu Tyr Asn Trp Asn Leu Pro Pro Arg Arg Ile Leu
   420 425 430
   Phe Ile Lys His Phe Ile Ile Tyr Ser Ile Pro Arg Tyr Val Arg Asp
   435 440 445
   Leu Lys Ile Gln Ile Glu Met Glu Lys Lys Val Val Phe Ser Thr Ile
   450 455 460
   Ser Leu Gly Lys Cys Ser Val Leu Asp Asn Ile Thr Thr Asp Lys Ile
   465 470 475 480
   Leu Ile Asp Val Phe Asp Gly Pro Pro Leu Tyr Asp Asp Val Lys Val
   485 490 495
   Gln Phe Phe Tyr Ser Asn Leu Pro Thr Tyr Tyr Asp Asn Cys Ser Phe
   500 505 510
   Tyr Phe Trp Leu His Thr Ser Phe Ile Glu Asn Asn Arg Leu Tyr Leu
   515 520 525
   Pro Lys Asn Glu Leu Asp Asn Leu His Lys Gln Lys Ala Arg Arg Ile
   530 535 540
   Tyr Pro Ser Asp Phe Ala Val Glu Ile Leu Phe Gly Glu Lys Met Thr
   545 550 555 560
   Ser Ser Asp Val Val Ala Gly Ser Asp
   565
<210> 27
   <211> 21
   <212> DNA
   <213> Künstliche Sequenz
<400> 27
   tggatgtcac tctcatcctt g 21
<210> 28
   <211> 21
   <212> DNA
   <213> Künstliche Sequenz
<400> 28
   ccatagttcc tgttctatct g 21
<210> 54
   <211> 1550
   <212> DNA
   <213> Homo sapiens
<400> 54
   atgaatgaaa gtcctgatcc gactgacctg gcgggagtca tcattgagct cggccccaat    60
   gacagtccac agacaagtga atttaaagga gcaaccgagg aggcacctgc gaaagaaagc   120
   ccacacacaa gtgaatttaa aggagcagcc cgggtgtcac ctatcagtga aagtgtgtta   180
   gcacgacttt ccaagtttga agttgaagat gctgaaaatg ttgcttcata tgacagcaag   240
   attaagaaaa ttgtgcattc aattgtatca tcctttgcat ttggactatt tggagttttc   300
   ctggtcttac tggatgtcac tctcatcctt gccgacctaa ttttcactga cagcaaactt   360
   tatattcctt tggagtatcg ttctatttct ctagctattg ccttattttt tctcatggat   420
   gttcttcttc gagtatttgt agaaaggaga cagcagtatt tttctgactt atttaacatt   480
   ttagatactg ccattattgt gattcttctg ctggttgatg tcgtttacat tttttttgac   540
   attaagttgc ttaggaatat tcccagatgg acacatttac ttcgacttct acgacttatt   600
   attctgttaa gaatttttca tctgtttcat caaaaaagac aacttgaaaa gctgataaga   660
   aggcgggttt cagaaaacaa aaggcgatac acaagggatg gatttgacct agacctcact   720
   tacgttacag aacgtattat tgctatgtca tttccatctt ctggaaggca gtctttctat   780
   agaaatccaa tcaaggaagt tgtgcggttt ctagataaga aacaccgaaa ccactatcga   840
   gtctacaatc tatgcagtga aagagcttac gatcctaagc acttccataa tagggtcgtt   900
   agaatcatga ttgatgatca taatgtcccc actctacatc agatggtggt tttcaccaag   960
   gaagtaaatg agtggatggc tcaagatctt gaaaacatcg tagcgattca ctgtaaagga  1020
   ggcacagata gaacaggaac tatggtttgt gccttcctta ttgcctctga aatatgttca  1080
   actgcaaagg aaagcctgta ttattttgga gaaaggcgaa cagataaaac ccacagcgaa  1140
   aaatttcagg gagtagaaac tccttctcag gttatgtacg tgatctaaaa atccaaatag  1200
   aaatggagaa aaaggttgtc ttttccacta tttcattagg aaaatgttcg gtacttgata  1260
   acattacaac agacaaaata ttaattgatg tattcgacgg tccacctctg tatgatgatg  1320
   tgaaagtgca gtttttctat tcgaatcttc ctacatacta tgacaattgc tcattttact  1380
   tctggttgca cacatctttt attgaaaata acaggcttta tctaccaaaa aatgaattgg  1440
   ataatctaca taaacaaaaa gcacggagaa tttatccatc agattttgcc gtggagatac  1500
   tttttggcga gaaaatgact tccagtgatg ttgtagctgg atccgattaa             1550
<210> 55
   <211> 1407
   <212> DNA
   <213> Homo sapiens
<400> 55
   atgaatgaaa gtcctgatcc gactgacctg gcgggagtca tcattgagct cggccccaat 60
   gacagtccac agacaagtga atttaaagga gcaaccgagg aggcacctgc gaaagaaagc 120
   ccacacacaa gtgaatttaa aggagcagcc cgggtgtcac ctatcagtga aagtgtgtta 180
   gcacgacttt ccaagtttga agttgaagat gctgaaaatg ttgcttcata tgacagcaag 240
   attaagaaaa ttgtgcattc aattgtatca tcctttgcat ttggactatt tggagttttc 300
   ctggtcttac tggatgtcac tctcatcctt gccgacctaa ttttcactga cagcaaactt 360
   tatattcctt tggagtatcg ttctatttct ctagctattg ccttattttt tctcatggat 420
   gttcttcttc gagtatttgt agaaaggaga cagcagtatt tttctgactt atttaacatt 480
   ttagatactg ccattattgt gattcttctg ctggttgatg tcgtttacat tttttttgac 540
   attaagttgc ttaggaatat tcccagatgg acacatttac ttcgacttct acgacttatt 600
   attctgttaa gaatttttca tctgtttcat caaaaaagac aacttgaaaa gctgataaga 660
   aggcgggttt cagaaaacaa aaggcgatac acaagggatg gatttgacct agacctcact 720
   tacgttacag aacgtattat tgctatgtca tttccatctt ctggaaggca gtctttctat 780
   agaaatccaa tcaaggaagt tgtgcggttt ctagataaga aacaccgaaa ccactatcga 840
   gtctacaatc tatgcagtga aagagcttac gatcctaagc acttccataa tagggtcgtt 900
   agaatcatga ttgatgatca taatgtcccc actctacatc agatggtggt tttcaccaag 960
   gaagtaaatg agtggatggc tcaagatctt gaaaacatcg tagcgattca ctgtaaagga 1020
   ggcacaggtt atgtacgtga tctaaaaatc caaatagaaa tggagaaaaa ggttgtcttt 1080
   tccactattt cattaggaaa atgttcggta cttgataaca ttacaacaga caaaatatta 1140
   attgatgtat tcgacggtcc acctctgtat gatgatgtga aagtgcagtt tttctattcg 1200
   aatcttccta catactatga caattgctca ttttacttct ggttgcacac atcttttatt 1260
   gaaaataaca ggctttatct accaaaaaat gaattggata atctacataa acaaaaagca 1320
   cggagaattt atccatcaga ttttgccgtg gagatacttt ttggcgagaa aatgacttcc 1380
   agtgatgttg tagctggatc cgattaa 1407
<210> 56
   <211> 1413
   <212> DNA
   <213> Homo sapiens
<400> 56
   atgaatgaaa gtcctgatcc gactgacctg gcgggagtca tcattgagct cggccccaat      60
   gacagtccac agacaagtga atttaaagga gcaaccgagg aggcacctgc gaaagaaagt     120
   gtgttagcac gactttccaa gtttgaagtt gaagatgctg aaaatgttgc ttcatatgac     180
   agcaagatta agaaaattgt gcattcaatt gtatcatcct ttgcatttgg actatttgga     240
   gttttcctgg tcttactgga tgtcactctc atccttgccg acctaatttt cactgacagc     300
   aaactttata ttcctttgga gtatcgttct atttctctag ctattgcctt attttttctc      360
   atggatgttc ttcttcgagt atttgtagaa aggagacagc agtatttttc tgacttattt     420
   aacattttag atactgccat tattgtgatt cttctgctgg ttgatgtcgt ttacattttt     480
   tttgacatta agttgcttag gaatattccc agatggacac atttacttcg acttctacga     540
   cttattattc tgttaagaat ttttcatctg tttcatcaaa aaagacaact tgaaaagctg     600
   ataagaaggc gggtttcaga aaacaaaagg cgatacacaa gggatggatt tgacctagac     660
   ctcacttacg ttacagaacg tattattgct atgtcatttc catcttctgg aaggcagtct     720
   ttctatagaa atccaatcaa ggaagttgtg cggtttctag ataagaaaca ccgaaaccac     780
   tatcgagtct acaatctatg cagtgaaaga gcttacgatc ctaagcactt ccataatagg     840
   gtcgttagaa tcatgattga tgatcataat gtccccactc tacatcagat ggtggttttc     900
   accaaggaag taaatgagtg gatggctcaa gatcttgaaa acatcgtagc gattcactgt     960
   aaaggaggca cagatagaac aggaactatg gtttgtgcct tccttattgc ctctgaaata    1020
   tgttcaactg caaaggaaag cctgtattat tttggagaaa ggcgaacaga taaaacccac    1080
   agcgaaaaat ttcagggagt agaaactcct tctgtacttg ataacattac aacagacaaa    1140
   atattaattg atgtattcga cggtccacct ctgtatgatg atgtgaaagt gcagtttttc    1200
   tattcgaatc ttcctacata ctatgacaat tgctcatttt acttctggtt gcacacatct    1260
   tttattgaaa ataacaggct ttatctacca aaaaatgaat tggataatct acataaacaa    1320
   aaagcacgga gaatttatcc atcagatttt gccgtggaga tactttttgg cgagaaaatg    1380
   acttccagtg atgttgtagc tggatccgat taa                                1413
<210> 57
   <211> 1353
   <212> DNA
   <213> Homo sapiens
<400> 57
   atgaatgaaa gtcctgatcc gactgacctg gcgggagtca tcattgagct cggccccaat      60
   gacagtccac agacaagtga atttaaagga gcaaccgagg aggcacctgc gaaagaaagt     120
   gtgttagcac gactttccaa gtttgaagtt gaagatgctg aaaatgttgc ttcatatgac     180
   agcaagatta agaaaattgt gcattcaatt gtatcatcct ttgcatttgg actatttgga     240
   gttttcctgg tcttactgga tgtcactctc atccttgccg acctaatttt cactgacagc     300
   aaactttata ttcctttgga gtatcgttct atttctctag ctattgcctt attttttctc      360
   atggatgttc ttcttcgagt atttgtagaa aggagacagc agtatttttc tgacttattt     420
   aacattttag atactgccat tattgtgatt cttctgctgg ttgatgtcgt ttacattttt     480
   tttgacatta agttgcttag gaatattccc agatggacac atttacttcg acttctacga     540
   cttattattc tgttaagaat ttttcatctg tttcatcaaa aaagacaact tgaaaagctg     600
   ataagaaggc gggtttcaga aaacaaaagg cgatacacaa gggatggatt tgacctagac     660
   ctcacttacg ttacagaacg tattattgct atgtcatttc catcttctgg aaggcagtct     720
   ttctatagaa atccaatcaa ggaagttgtg cggtttctag ataagaaaca ccgaaaccac     780
   tatcgagtct acaatctatg cagtgaaaga gcttacgatc ctaagcactt ccataatagg     840
   gtcgttagaa tcatgattga tgatcataat gtccccactc tacatcagat ggtggttttc     900
   accaaggaag taaatgagtg gatggctcaa gatcttgaaa acatcgtagc gattcactgt     960
   aaaggaggca caggttatgt acgtgatcta aaaatccaaa tagaaatgga gaaaaaggtt    1020
   gtcttttcca ctatttcatt aggaaaatgt tcggtacttg ataacattac aacagacaaa    1080
   atattaattg atgtattcga cggtccacct ctgtatgatg atgtgaaagt gcagtttttc    1140
   tattcgaatc ttcctacata ctatgacaat tgctcatttt acttctggtt gcacacatct    1200
   tttattgaaa ataacaggct ttatctacca aaaaatgaat tggataatct acataaacaa    1260
   aaagcacgga gaatttatcc atcagatttt gccgtggaga tactttttgg cgagaaaatg    1320
   acttccagtg atgttgtagc tggatccgat taa                                 1353
<210> 58
   <211> 395
   <212> PRT
   <213> Homo sapiens
<400> 58
   Met Asn Glu Ser Pro Asp Pro Thr Asp Leu Ala Gly Val Ile Ile Glu
   1 5 10 15
   Leu Gly Pro Asn Asp Ser Pro Gln Thr Ser Glu Phe Lys Gly Ala Thr
   20 25 30
   Glu Glu Ala Pro Ala Lys Glu Ser Pro His Thr Ser Glu Phe Lys Gly
   35 40 45
   Ala Ala Arg Val Ser Pro Ile Ser Glu Ser Val Leu Ala Arg Leu Ser
   50 55 60
   Lys Phe Glu Val Glu Asp Ala Glu Asn Val Ala Ser Tyr Asp Ser Lys
   65 70 75 80
   Ile Lys Lys Ile Val His Ser Ile Val Ser Ser Phe Ala Phe Gly Leu
   85 90 95
   Phe Gly Val Phe Leu Val Leu Leu Asp Val Thr Leu Ile Leu Ala Asp
   100 105 110
   Leu Ile Phe Thr Asp Ser Lys Leu Tyr Ile Pro Leu Glu Tyr Arg Ser
   115 120 125
   Ile Ser Leu Ala Ile Ala Leu Phe Phe Leu Met Asp Val Leu Leu Arg
   130 135 140
   Val Phe Val Glu Arg Arg Gln Gln Tyr Phe Ser Asp Leu Phe Asn Ile
   145 150 155 160
   Leu Asp Thr Ala Ile Ile Val Ile Leu Leu Leu Val Asp Val Val Tyr
   165 170 175
   Ile Phe Phe Asp Ile Lys Leu Leu Arg Asn Ile Pro Arg Trp Thr His
   180 185 190
   Leu Leu Arg Leu Leu Arg Leu Ile Ile Leu Leu Arg Ile Phe His Leu
   195 200 205
   Phe His Gln Lys Arg Gln Leu Glu Lys Leu Ile Arg Arg Arg Val Ser
   210 215 220
   Glu Asn Lys Arg Arg Tyr Thr Arg Asp Gly Phe Asp Leu Asp Leu Thr
   225 230 235 240
   Tyr Val Thr Glu Arg Ile Ile Ala Met Ser Phe Pro Ser Ser Gly Arg
   245 250 255
   Gln Ser Phe Tyr Arg Asn Pro Ile Lys Glu Val Val Arg Phe Leu Asp
   260 265 270
   Lys Lys His Arg Asn His Tyr Arg Val Tyr Asn Leu Cys Ser Glu Arg
   275 280 285
   Ala Tyr Asp Pro Lys His Phe His Asn Arg Val Val Arg Ile Met Ile
   290 295 300
   Asp Asp His Asn Val Pro Thr Leu His Gln Met Val Val Phe Thr Lys
   305 310 315 320
   Glu Val Asn Glu Trp Met Ala Gln Asp Leu Glu Asn Ile Val Ala Ile
   325 330 335
   His Cys Lys Gly Gly Thr Asp Arg Thr Gly Thr Met Val Cys Ala Phe
   340 345 350
   Leu Ile Ala Ser Glu Ile Cys Ser Thr Ala Lys Glu Ser Leu Tyr Tyr
   355 360 365
   Phe Gly Glu Arg Arg Thr Asp Lys Thr His Ser Glu Lys Phe Gln Gly
   370 375 380
   Val Glu Thr Pro Ser Gln Val Met Tyr Val Ile
   385 390 395
<210> 59
   <211> 468
   <212> PRT
   <213> Homo sapiens
<400> 59
   Met Asn Glu Ser Pro Asp Pro Thr Asp Leu Ala Gly Val Ile Ile Glu
   1 5 10 15
   Leu Gly Pro Asn Asp Ser Pro Gln Thr Ser Glu Phe Lys Gly Ala Thr
   20 25 30
   Glu Glu Ala Pro Ala Lys Glu Ser Pro His Thr Ser Glu Phe Lys Gly
   35 40 45
   Ala Ala Arg Val Ser Pro Ile Ser Glu Ser Val Leu Ala Arg Leu Ser
   50 55 60
   Lys Phe Glu Val Glu Asp Ala Glu Asn Val Ala Ser Tyr Asp Ser Lys
   65 70 75 80
   Ile Lys Lys Ile Val His Ser Ile Val Ser Ser Phe Ala Phe Gly Leu
   85 90 95
   Phe Gly Val Phe Leu Val Leu Leu Asp Val Thr Leu Ile Leu Ala Asp
   100 105 110
   Leu Ile Phe Thr Asp Ser Lys Leu Tyr Ile Pro Leu Glu Tyr Arg Ser
   115 120 125
   Ile Ser Leu Ala Ile Ala Leu Phe Phe Leu Met Asp Val Leu Leu Arg
   130 135 140
   Val Phe Val Glu Arg Arg Gln Gln Tyr Phe Ser Asp Leu Phe Asn Ile
   145 150 155 160
   Leu Asp Thr Ala Ile Ile Val Ile Leu Leu Leu Val Asp Val Val Tyr
   165 170 175
   Ile Phe Phe Asp Ile Lys Leu Leu Arg Asn Ile Pro Arg Trp Thr His
   180 185 190
   Leu Leu Arg Leu Leu Arg Leu Ile Ile Leu Leu Arg Ile Phe His Leu
   195 200 205
   Phe His Gln Lys Arg Gln Leu Glu Lys Leu Ile Arg Arg Arg Val Ser
   210 215 220
   Glu Asn Lys Arg Arg Tyr Thr Arg Asp Gly Phe Asp Leu Asp Leu Thr
   225 230 235 240
   Tyr Val Thr Glu Arg Ile Ile Ala Met Ser Phe Pro Ser Ser Gly Arg
   245 250 255
   Gln Ser Phe Tyr Arg Asn Pro Ile Lys Glu Val Val Arg Phe Leu Asp
   260 265 270
   Lys Lys His Arg Asn His Tyr Arg Val Tyr Asn Leu Cys Ser Glu Arg
   275 280 285
   Ala Tyr Asp Pro Lys His Phe His Asn Arg Val Val Arg Ile Met Ile
   290 295 300
   Asp Asp His Asn Val Pro Thr Leu His Gln Met Val Val Phe Thr Lys
   305 310 315 320
   Glu Val Asn Glu Trp Met Ala Gln Asp Leu Glu Asn Ile Val Ala Ile
   325 330 335
   His Cys Lys Gly Gly Thr Gly Tyr Val Arg Asp Leu Lys Ile Gln Ile
   340 345 350
   Glu Met Glu Lys Lys Val Val Phe Ser Thr Ile Ser Leu Gly Lys Cys
   355 360 365
   Ser Val Leu Asp Asn Ile Thr Thr Asp Lys Ile Leu Ile Asp Val Phe
   370 375 380
   Asp Gly Pro Pro Leu Tyr Asp Asp Val Lys Val Gln Phe Phe Tyr Ser
   385 390 395 400
   Asn Leu Pro Thr Tyr Tyr Asp Asn Cys Ser Phe Tyr Phe Trp Leu His
   405 410 415
   Thr Ser Phe Ile Glu Asn Asn Arg Leu Tyr Leu Pro Lys Asn Glu Leu
   420 425 430
   Asp Asn Leu His Lys Gln Lys Ala Arg Arg Ile Tyr Pro Ser Asp Phe
   435 440 445
   Ala Val Glu Ile Leu Phe Gly Glu Lys Met Thr Ser Ser Asp Val Val
   450 455 460
   Ala Gly Ser Asp
   465
<210> 60
   <211> 470
   <212> PRT
   <213> Homo sapiens
<400> 60
   Met Asn Glu Ser Pro Asp Pro Thr Asp Leu Ala Gly Val Ile Ile Glu
   1 5 10 15
   Leu Gly Pro Asn Asp Ser Pro Gln Thr Ser Glu Phe Lys Gly Ala Thr
   20 25 30
   Glu Glu Ala Pro Ala Lys Glu Ser Val Leu Ala Arg Leu Ser Lys Phe
   35 40 45
   Glu Val Glu Asp Ala Glu Asn Val Ala Ser Tyr Asp Ser Lys Ile Lys
   50 55 60
   Lys Ile Val His Ser Ile Val Ser Ser Phe Ala Phe Gly Leu Phe Gly
   65 70 75 80
   Val Phe Leu Val Leu Leu Asp Val Thr Leu Ile Leu Ala Asp Leu Ile
   85 90 95
   Phe Thr Asp Ser Lys Leu Tyr Ile Pro Leu Glu Tyr Arg Ser Ile Ser
   100 105 110
   Leu Ala Ile Ala Leu Phe Phe Leu Met Asp Val Leu Leu Arg Val Phe
   115 120 125
   Val Glu Arg Arg Gln Gln Tyr Phe Ser Asp Leu Phe Asn Ile Leu Asp
   130 135 140
   Thr Ala Ile Ile Val Ile Leu Leu Leu Val Asp Val Val Tyr Ile Phe
   145 150 155 160
   Phe Asp Ile Lys Leu Leu Arg Asn Ile Pro Arg Trp Thr His Leu Leu
   165 170 175
   Arg Leu Leu Arg Leu Ile Ile Leu Leu Arg Ile Phe His Leu Phe His
   180 185 190
   Gln Lys Arg Gln Leu Glu Lys Leu Ile Arg Arg Arg Val Ser Glu Asn
   195 200 205
   Lys Arg Arg Tyr Thr Arg Asp Gly Phe Asp Leu Asp Leu Thr Tyr Val
   210 215 220
   Thr Glu Arg Ile Ile Ala Met Ser Phe Pro Ser Ser Gly Arg Gln Ser
   225 230 235 240
   Phe Tyr Arg Asn Pro Ile Lys Glu Val Val Arg Phe Leu Asp Lys Lys
   245 250 255
   His Arg Asn His Tyr Arg Val Tyr Asn Leu Cys Ser Glu Arg Ala Tyr
   260 265 270
   Asp Pro Lys His Phe His Asn Arg Val Val Arg Ile Met Ile Asp Asp
   275 280 285
   His Asn Val Pro Thr Leu His Gln Met Val Val Phe Thr Lys Glu Val
   290 295 300
   Asn Glu Trp Met Ala Gln Asp Leu Glu Asn Ile Val Ala Ile His Cys
   305 310 315 320
   Lys Gly Gly Thr Asp Arg Thr Gly Thr Met Val Cys Ala Phe Leu Ile
   325 330 335
   Ala Ser Glu Ile Cys Ser Thr Ala Lys Glu Ser Leu Tyr Tyr Phe Gly
   340 345 350
   Glu Arg Arg Thr Asp Lys Thr His Ser Glu Lys Phe Gln Gly Val Glu
   355 360 365
   Thr Pro Ser Val Leu Asp Asn Ile Thr Thr Asp Lys Ile Leu Ile Asp
   370 375 380
   Val Phe Asp Gly Pro Pro Leu Tyr Asp Asp Val Lys Val Gln Phe Phe
   385 390 395 400
   Tyr Ser Asn Leu Pro Thr Tyr Tyr Asp Asn Cys Ser Phe Tyr Phe Trp
   405 410 415
   Leu His Thr Ser Phe Ile Glu Asn Asn Arg Leu Tyr Leu Pro Lys Asn
   420 425 430
   Glu Leu Asp Asn Leu His Lys Gln Lys Ala Arg Arg Ile Tyr Pro Ser
   435 440 445
   Asp Phe Ala Val Glu Ile Leu Phe Gly Glu Lys Met Thr Ser Ser Asp
   450 455 460
   Val Val Ala Gly Ser Asp
   465 470
<210> 61
   <211> 450
   <212> PRT
   <213> Homo sapiens
<400> 61
   Met Asn Glu Ser Pro Asp Pro Thr Asp Leu Ala Gly Val Ile Ile Glu
   1 5 10 15
   Leu Gly Pro Asn Asp Ser Pro Gln Thr Ser Glu Phe Lys Gly Ala Thr
   20 25 30
   Glu Glu Ala Pro Ala Lys Glu Ser Val Leu Ala Arg Leu Ser Lys Phe
   35 40 45
   Glu Val Glu Asp Ala Glu Asn Val Ala Ser Tyr Asp Ser Lys Ile Lys
   50 55 60
   Lys Ile Val His Ser Ile Val Ser Ser Phe Ala Phe Gly Leu Phe Gly
   65 70 75 80
   Val Phe Leu Val Leu Leu Asp Val Thr Leu Ile Leu Ala Asp Leu Ile
   85 90 95
   Phe Thr Asp Ser Lys Leu Tyr Ile Pro Leu Glu Tyr Arg Ser Ile Ser
   100 105 110
   Leu Ala Ile Ala Leu Phe Phe Leu Met Asp Val Leu Leu Arg Val Phe
   115 120 125
   Val Glu Arg Arg Gln Gln Tyr Phe Ser Asp Leu Phe Asn Ile Leu Asp
   130 135 140
   Thr Ala Ile Ile Val Ile Leu Leu Leu Val Asp Val Val Tyr Ile Phe
   145 150 155 160
   Phe Asp Ile Lys Leu Leu Arg Asn Ile Pro Arg Trp Thr His Leu Leu
   165 170 175
   Arg Leu Leu Arg Leu Ile Ile Leu Leu Arg Ile Phe His Leu Phe His
   180 185 190
   Gln Lys Arg Gln Leu Glu Lys Leu Ile Arg Arg Arg Val Ser Glu Asn
   195 200 205
   Lys Arg Arg Tyr Thr Arg Asp Gly Phe Asp Leu Asp Leu Thr Tyr Val
   210 215 220
   Thr Glu Arg Ile Ile Ala Met Ser Phe Pro Ser Ser Gly Arg Gln Ser
   225 230 235 240
   Phe Tyr Arg Asn Pro Ile Lys Glu Val Val Arg Phe Leu Asp Lys Lys
   245 250 255
   His Arg Asn His Tyr Arg Val Tyr Asn Leu Cys Ser Glu Arg Ala Tyr
   260 265 270
   Asp Pro Lys His Phe His Asn Arg Val Val Arg Ile Met Ile Asp Asp
   275 280 285
   His Asn Val Pro Thr Leu His Gln Met Val Val Phe Thr Lys Glu Val
   290 295 300
   Asn Glu Trp Met Ala Gln Asp Leu Glu Asn Ile Val Ala Ile His Cys
   305 310 315 320
   Lys Gly Gly Thr Gly Tyr Val Arg Asp Leu Lys Ile Gln Ile Glu Met
   325 330 335
   Glu Lys Lys Val Val Phe Ser Thr Ile Ser Leu Gly Lys Cys Ser Val
   340 345 350
   Leu Asp Asn Ile Thr Thr Asp Lys Ile Leu Ile Asp Val Phe Asp Gly
   355 360 365
   Pro Pro Leu Tyr Asp Asp Val Lys Val Gln Phe Phe Tyr Ser Asn Leu
   370 375 380
   Pro Thr Tyr Tyr Asp Asn Cys Ser Phe Tyr Phe Trp Leu His Thr Ser
   385 390 395 400
   Phe Ile Glu Asn Asn Arg Leu Tyr Leu Pro Lys Asn Glu Leu Asp Asn
   405 410 415
   Leu His Lys Gln Lys Ala Arg Arg Ile Tyr Pro Ser Asp Phe Ala Val
   420 425 430
   Glu Ile Leu Phe Gly Glu Lys Met Thr Ser Ser Asp Val Val Ala Gly
   435 440 445
   Ser Asp
   450
<210> 81
   <211> 14
   <212> PRT
   <213> Homo sapiens
<400> 81
   Phe Thr Asp Ser Lys Leu Tyr Ile Pro Leu Glu Tyr Arg Ser
   1 5 10
<210> 82
   <211> 13
   <212> PRT
   <213> Homo sapiens
<400> 82
   Phe Asp Ile Lys Leu Leu Arg Asn Ile Pro Arg Trp Thr
   1 5 10
<210> 83
   <211> 15
   <212> PRT
   <213> Homo sapiens

## Patentansprüche

1. Verwendung
(i) einer Polynukleotid-Sonde, die spezifisch mit einer Nukleinsäure, die für ein Tumor-assoziiertes Antigen kodiert, hybridisiert, und/oder
(ii) eines Antikörpers, der spezifisch an ein Tumor-assoziiertes Antigen bindet, und/oder
(iii) eines Proteins oder Peptids, das spezifisch an einen Antikörper gegen ein Tumor-assoziiertes Antigen bindet,
zur Herstellung einer pharmazeutischen Zusammensetzung zur Diagnose oder Überwachung einer Krebserkrankung, die sich durch die Expression des Tumor-assoziierten Antigens auszeichnet, in einer aus einem Patienten isolierten biologischen Probe, wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, und 54-57 ausgewählt ist,
und
(b) einer Nukleinsäure, die zu der Nukleinsäure unter (a) mindestens 98% Sequenzidentität aufweist.

2. Verwendung eines Antikörpers, der an ein Tumor-assoziiertes Antigen bindet und mit einem diagnostischen Mittel gekoppelt ist, zur Herstellung einer pharmazeutischen Zusammensetzung zur Diagnose oder Überwachung einer Krebserkrankung, die sich durch die Expression des Tumor-assoziierten Antigens auszeichnet, wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, und 54-57 ausgewählt ist,
und
(b) einer Nukleinsäure, die zu der Nukleinsäure unter (a) mindestens 98% Sequenzidentität aufweist.

3. Verwendung nach Anspruch 1 oder 2, wobei der Antikörper, der an ein Tumor-assoziiertes Antigen bindet, ein monoklonaler, chimärer oder humanisierter Antikörper oder ein Fragment eines Antikörpers ist.

4. Verwendung nach einem der Ansprüche 1-3, wobei das Tumor-assoziierte Antigen eine Aminosäuresequenz umfasst, die aus der Gruppe bestehend aus SEQ ID NOs: 22-24, 58-61, 81, und 82 ausgewählt ist.

5. Antikörper, der spezifisch an ein Tumor-assoziiertes Antigen bindet, wobei das Tumor-assoziierte Antigen von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, und 54-57 ausgewählt ist,
und
(b) einer Nukleinsäure, die zu der Nukleinsäure unter (a) mindestens 98% Sequenzidentität aufweist,
zur Verwendung in einem Verfahren zur Diagnose einer Krebserkrankung, die sich durch die Expression des Tumor-assoziierten Antigens auszeichnet.

6. Antikörper nach Anspruch 5, wobei das Protein oder Polypeptid eine Aminosäuresequenz umfasst, die aus der Gruppe ausgewählt ist, bestehend aus SEQ ID NOs: 22-24, 58-61, 81, und 82.

7. Antikörper nach Anspruch 5 oder 6, wobei der Antikörper ein monoklonaler, chimärer oder humanisierter Antikörper oder ein Fragment eines Antikörpers ist.

8. Antikörper nach einem der Ansprüche 5-7, wobei der Antikörper an ein diagnostisches Mittel gekoppelt ist.

9. Antikörper nach Anspruch 8, wobei das diagnostische Mittel ein Toxin ist.

10. Verwendung eines Kits zur Diagnose einer Krebserkrankung, die sich durch die Expression eines Tumor-assoziierten Antigens auszeichnet, umfassend Mittel zum Nachweis
(i) der Nukleinsäure, die für das Tumor-assoziierte Antigen kodiert,
(ii) des Tumor-assoziierten Antigens,
(iii) von Antikörpern, die an das Tumor-assoziierte Antigen binden, und/oder
(iv) von T-Zellen, die für einen Komplex zwischen dem Tumor-assoziierten Antigen oder einem Teil davon, der mindestens 6 aufeinander folgende Aminosäuren umfasst, und einem MHC-Molekül spezifisch sind,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, und 54-57 ausgewählt ist,
und
(b) einer Nukleinsäure, die zu der Nukleinsäure unter (a) mindestens 98% Sequenzidentität aufweist.

11. Verwendung nach Anspruch 10, wobei die Mittel zum Nachweis der Nukleinsäure, die für das Tumor-assoziierte Antigen kodiert, Nukleinsäuremoleküle für die selektive Amplifikation der Nukleinsäure sind.

12. Verwendung eines Antikörpers, der an TPTE bindet und mit einem diagnostischen Mittel gekoppelt ist, zur Herstellung einer pharmazeutischen Zusammensetzung zur Diagnose oder Überwachung eines metastasierenden Tumors, der sich durch die Expression von TPTE auszeichnet, wobei das TPTE eine Sequenz aufweist, die von einer Nukleinsäure kodiert wird, bestehend aus:
(a) einer Nukleinsäure, die die Nukleinsäuresequenz gemäß SEQ ID NO: 19 umfasst,
und
(b) einer Nukleinsäure, die zu der Nukleinsäure unter (a) mindestens 98% Sequenzidentität aufweist.

13. Verwendung nach Anspruch 12, wobei der Antikörper ein monoklonaler, chimärer oder humanisierter Antikörper oder ein Fragment eines Antikörpers ist.

14. *In vitro* Verfahren zur Diagnose einer Krebserkrankung, die sich durch die Expression eines Tumor-assoziierten Antigens auszeichnet, umfassend
(i) den Nachweis einer Nukleinsäure, die für ein Tumor-assoziiertes Antigen kodiert, und/oder
(ii) den Nachweis eines Tumor-assoziierten Antigens und/oder
(iii) den Nachweis eines Antikörpers gegen ein Tumor-assoziiertes Antigen und/oder
(iv) den Nachweis von cytotoxischen oder Helfer-T-Zellen, die für ein Tumor-assoziiertes Antigen oder einen Teil davon, der mindestens 6 aufeinander folgende Aminosäuren umfasst, spezifisch sind, in einer aus einem Patienten isolierten biologischen Probe, wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, und 54-57 ausgewählt ist,
und
(b) einer Nukleinsäure, die zu der Nukleinsäure unter (a) mindestens 98% Sequenzidentität aufweist.

15. Verfahren nach Anspruch 14, wobei der Nachweis
(i) die Kontaktierung der biologischen Probe mit einem Mittel, das spezifisch an die Nukleinsäure, die für das Tumor-assoziierte Antigen kodiert, an das Tumor-assoziierte Antigen, an den Antikörper oder an die cytotoxischen oder Helfer-T-Zellen bindet, und
(ii) den Nachweis der Komplexbildung zwischen dem Mittel und der Nukleinsäure, dem Tumor-assoziierten Antigen, dem Antikörper oder den cytotoxischen oder Helfer-T-Zellen umfasst.

16. Verfahren nach Anspruch 14 oder 15, wobei der Nachweis mit dem Nachweis in einer vergleichbaren normalen biologischen Probe verglichen wird.

17. *In vitro* Verfahren zur Bestimmung der Regression, des Verlaufs oder des Ausbruchs einer Krebserkrankung, die sich durch die Expression eines Tumor-assoziierten Antigens auszeichnet, umfassend die Überwachung einer Probe aus einem Patienten, der Krebs aufweist oder in Verdacht steht, an Krebs zu erkranken, in Bezug auf einen oder mehrere Parameter, ausgewählt aus der Gruppe, bestehend aus:
(i) der Menge einer Nukleinsäure, die für das Tumor-assoziiertes Antigen kodiert,
(ii) der Menge des Tumor-assoziierten Antigens,
(iii) der Menge an Antikörpern, die an das Tumor-assoziiertes Antigen binden, und
(iv) der Menge an cytolytischen oder Cytokin-ausschüttenden T-Zellen, die für einen Komplex zwischen dem Tumor-assoziierten Antigen oder einem Teil davon, der mindestens 6 aufeinander folgende Aminosäuren umfasst, und einem MHC-Molekül spezifisch sind, wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, und 54-57 ausgewählt ist,
und
(b) einer Nukleinsäure, die zu der Nukleinsäure unter (a) mindestens 98% Sequenzidentität aufweist.

18. Verfahren nach Anspruch 17, wobei das Verfahren die Bestimmung des oder der Parameter zu einem ersten Zeitpunkt in einer ersten Probe und zu einem zweiten Zeitpunkt in einer weiteren Probe umfasst und durch einen Vergleich der beiden Proben der Verlauf der Erkrankung ermittelt wird.

19. Verfahren nach einem der Ansprüche 14-18, wobei der Nachweis der Nukleinsäure oder die Überwachung der Menge der Nukleinsäure mit einer Polynukleotid-Sonde erfolgt, die spezifisch mit der Nukleinsäure hybridisiert.

20. Verfahren nach Anspruch 19, wobei die Polynukleotid-Sonde eine Sequenz von 6-50 zusammenhängenden Nukleotiden aus der Nukleinsäure, die für das Tumor-assoziierte Antigen kodiert, umfasst.

21. Verfahren nach einem der Ansprüche 14-18, wobei der Nachweis der Nukleinsäure oder die Überwachung der Menge der Nukleinsäure durch selektive Amplifikation der Nukleinsäure erfolgt.

22. Verfahren nach einem der Ansprüche 14-18, wobei der Nachweis des Tumor-assoziierten Antigens oder die Überwachung der Menge des Tumor-assoziierten Antigens mit einem Antikörper erfolgt, der spezifisch an das Tumor-assoziierte Antigen bindet.

23. Verfahren nach einem der Ansprüche 14-18, wobei der Nachweis des Antikörpers oder die Überwachung der Menge an Antikörpern mit einem Protein oder Peptid erfolgt, das spezifisch an den Antikörper bindet.

24. Verfahren nach einem der Ansprüche 14-18, wobei der Nachweis der T-Zellen oder die Überwachung der Menge an T-Zellen mit einer Zelle erfolgt, die den Komplex zwischen dem Tumor-assoziierten Antigen oder dem Teil davon und einem MHC-Molekül präsentiert.

25. Verfahren nach einem der Ansprüche 19, 20, und 22-24, wobei die Polynukleotid-Sonde, der Antikörper, das Protein oder Peptid oder die Zelle nachweisbar markiert sind.

26. Verfahren nach Anspruch 25, wobei der nachweisbare Marker ein radioaktiver Marker oder ein Enzymmarker ist.

27. Verfahren nach einem der Ansprüche 14-26, wobei die Probe Körperflüssigkeit und/oder Körpergewebe umfasst.

28. Verfahren nach einem der Ansprüche 14-27, wobei das Tumor-assoziierte Antigen eine Aminosäuresequenz umfasst, die aus der Gruppe bestehend aus SEQ ID NOs: 22-24, 58-61, 81, und 82 ausgewählt ist.

## Claims

1. Use of
(i) a polynucleotide probe which hybridizes specifically to a nucleic acid which codes for a tumor-associated antigen and/or
(ii) an antibody binding specifically to a tumor-associated antigen and/or
(iii) a protein or peptide binding specifically to an antibody to a tumor-associated antigen
for the preparation of a pharmaceutical composition for diagnosing or monitoring a cancer disease **characterized by** expression of the tumor-associated antigen in a biological sample isolated from a patient,
said tumor-associated antigen having a sequence encoded by a nucleic acid which is selected from the group consisting of:
(a) a nucleic acid which comprises a nucleic acid sequence selected from the group consisting of SEQ ID NOs: 19-21 and 54-57, and
(b) a nucleic acid which has at least 98% sequence identity to the nucleic acid of (a).

2. Use of an antibody binding to a tumor-associated antigen and coupled to a diagnostic agent for the preparation of a pharmaceutical composition for diagnosing or monitoring a cancer disease **characterized by** expression of the tumor-associated antigen, said tumor-associated antigen having a sequence encoded by a nucleic acid which is selected from the group consisting of:
(a) a nucleic acid which comprises a nucleic acid sequence selected from the group consisting of SEQ ID NOs: 19-21 and 54-57, and
(b) a nucleic acid which has at least 98% sequence identity to the nucleic acid of (a).

3. The use of claim 1 or 2, wherein the antibody binding to a tumor-associated antigen is a monoclonal, chimeric or humanized antibody or a fragment of an antibody.

4. The use of any one of claims 1-3, wherein the tumor-associated antigen comprises an amino acid sequence selected from the group consisting of SEQ ID NOs: 22-24, 58-61, 81 and 82.

5. An antibody which binds specifically to a tumor-associated antigen, said tumor-associated antigen being encoded by a nucleic acid selected from the group consisting of:
(a) a nucleic acid which comprises a nucleic acid sequence selected from the group consisting of SEQ ID NOs: 19-21 and 54-57, and
(b) a nucleic acid which has at least 98% sequence identity to the nucleic acid of (a)
for use in a method of diagnosing a cancer disease **characterized by** expression of the tumor-associated antigen.

6. The antibody of claim 5, wherein the protein or polypeptide comprises an amino acid sequence selected from the group consisting of SEQ ID NOs: 22-24, 58-61, 81 and 82.

7. The antibody of claim 5 or 6, wherein the antibody is a monoclonal, chimeric or humanized antibody or a fragment of an antibody.

8. The antibody of any one of claims 5-7, wherein the antibody is coupled to a diagnostic agent.

9. The antibody of claim 8, wherein the diagnostic agent is a toxin.

10. Use of a kit for diagnosing a cancer disease **characterized by** expression of a tumor-associated antigen, which kit comprises agents for detection
(i) of the nucleic acid which codes for the tumor-associated antigen,
(ii) of the tumor-associated antigen,
(iii) of antibodies which bind to the tumor-associated antigen and/or
(iv) of T cells which are specific for a complex between the tumor-associated antigen or a part thereof comprising at least 6 contiguous amino acids and an MHC molecule,
said tumor-associated antigen having a sequence encoded by a nucleic acid which is selected from the group consisting of:
(a) a nucleic acid which comprises a nucleic acid sequence selected from the group consisting of SEQ ID NOs: 19-21 and 54-57, and
(b) a nucleic acid which has at least 98% sequence identity to the nucleic acid of (a).

11. The use of claim 10, wherein the agents for detection of the nucleic acid which codes for the tumor-associated antigen are nucleic acid molecules for selective amplification of said nucleic acid.

12. Use of an antibody binding to TPTE and coupled to a diagnostic agent for the preparation of a pharmaceutical composition for diagnosing or monitoring a metastasizing tumor **characterized by** expression of TPTE, said TPTE having a sequence encoded by a nucleic acid consisting of:
(a) a nucleic acid which comprises the nucleic acid sequence according to SEQ ID NO: 19, and
(b) a nucleic acid which has at least 98% sequence identity to the nucleic acid of (a).

13. The use of claim 12, wherein the antibody is a monoclonal, chimeric or humanized antibody or a fragment of an antibody.

14. An *in vitro* method of diagnosing a cancer disease **characterized by** expression of a tumor-associated antigen, which method comprises
(i) detection of a nucleic acid which codes for a tumor-associated antigen and/or
(ii) detection of a tumor-associated antigen and/or
(iii) detection of an antibody to a tumor-associated antigen and/or
(iv) detection of cytotoxic or T helper cells which are specific to a tumor-associated antigen or to a part thereof comprising at least 6 contiguous amino acids
in a biological sample isolated from a patient,
said tumor-associated antigen having a sequence encoded by a nucleic acid which is selected from the group consisting of:
(a) a nucleic acid which comprises a nucleic acid sequence selected from the group consisting of SEQ ID NOs: 19-21 and 54-57, and
(b) a nucleic acid which has at least 98% sequence identity to the nucleic acid of (a).

15. The method of claim 14, wherein the detection comprises
(i) contacting the biological sample with an agent which binds specifically to the nucleic acid coding for the tumor-associated antigen, to the tumor-associated antigen, to the antibody or to the cytotoxic or T helper cells, and
(ii) detecting the formation of a complex between the agent and the nucleic acid, the tumor-associated antigen, the antibody or the cytotoxic or T helper cells.

16. The method of claim 14 or 15, wherein the detection is compared to detection in a comparable normal biological sample.

17. An *in vitro* method for determining regression, course or onset of a cancer disease **characterized by** expression of a tumor-associated antigen, which method comprises monitoring a sample from a patient who has cancer or is suspected of falling ill with cancer with respect to one or more parameters selected from the group consisting of:
(i) the amount of nucleic acid which codes for the tumor-associated antigen,
(ii) the amount of the tumor-associated antigen,
(iii) the amount of antibodies which bind to the tumor-associated antigen and
(iv) the amount of cytolytic or cytokine-releasing T cells which are specific for a complex between the tumor-associated antigen or a part thereof comprising at least 6 contiguous amino acids and an MHC molecule,
said tumor-associated antigen having a sequence encoded by a nucleic acid which is selected from the group consisting of:
(a) a nucleic acid which comprises a nucleic acid sequence selected from the group consisting of SEQ ID NOs: 19-21 and 54-57, and
(b) a nucleic acid which has at least 98% sequence identity to the nucleic acid of (a).

18. The method of claim 17, which comprises determining the parameter(s) in a first sample at a first point in time and in a further sample at a second point in time and in which the course of the disease is determined by comparing the two samples.

19. The method of any one of claims 14-18, wherein the nucleic acid is detected or the amount of the nucleic acid is monitored using a polynucleotide probe which hybridizes specifically to said nucleic acid.

20. The method of claim 19, wherein the polynucleotide probe comprises a sequence of 6-50 contiguous nucleotides of the nucleic acid coding for the tumor-associated antigen.

21. The method of any one of claims 14-18, wherein the nucleic acid is detected or the amount of the nucleic acid is monitored by selectively amplifying said nucleic acid.

22. The method of any one of claims 14-18, wherein the tumor-associated antigen is detected or the amount of the tumor-associated antigen is monitored using an antibody binding specifically to said tumor-associated antigen.

23. The method of any one of claims 14-18, wherein the antibody is detected or the amount of antibodies is monitored using a protein or peptide binding specifically to the antibody.

24. The method of any one of claims 14-18, wherein the T cells are detected or the amount of T cells is monitored using a cell presenting the complex between the tumor-associated antigen or the part thereof and an MHC molecule.

25. The method of any one of claims 19, 20 and 22-24, wherein the polynucleotide probe, the antibody, the protein or peptide or the cell is labeled in a detectable manner.

26. The method of claim 25, wherein the detectable marker is a radioactive marker or an enzymic marker.

27. The method of any one of claims 14-26, wherein the sample comprises body fluid and/or body tissue.

28. The method of any one of claims 14-27, wherein the tumor-associated antigen comprises an amino acid sequence selected from the group consisting of SEQ ID NOs: 22-24, 58-61, 81 and 82.

## Revendications

1. Utilisation
(i) d'une sonde polynucléotidique, qui hybride spécifiquement avec un acide nucléique qui code pour un antigène associé à une tumeur, et/ou
(ii) d'un anticorps, qui se lie spécifiquement à un antigène associé à une tumeur, et/ou
(iii) d'une protéine ou d'un peptide, qui se lie spécifiquement à un anticorps contre un antigène associé à une tumeur,
pour la préparation d'une composition pharmaceutique pour le diagnostic ou le suivi d'une affection cancéreuse, qui se **caractérise par** l'expression de l'antigène associé à une tumeur, dans un échantillon biologique isolé à partir d'un patient, tandis que
l'antigène associé à une tumeur présente une séquence qui est codée par un acide nucléique qui est choisi dans le groupe consistant en:
(a) un acide nucléique qui comporte une séquence d'acide nucléique qui est choisie dans le groupe consistant en SEQ ID NOs: 19-21, et 54-57,
et
(b) un acide nucléique qui présente vis-à-vis de l'acide nucléique sous (a) une identité de séquence d'au moins 98%.

2. Utilisation d'un anticorps qui se lie à un antigène associé à une tumeur et est couplé avec un moyen de diagnostic, pour la préparation d'une composition pharmaceutique pour le diagnostic ou le suivi d'une affection cancéreuse, qui se distingue par l'expression de l'antigène associé à une tumeur, tandis que l'antigène associé à une tumeur présente une séquence qui est codée par un acide nucléique qui est choisi dans le groupe consistant en:
(a) un acide nucléique qui comporte une séquence d'acide nucléique qui est choisie dans le groupe consistant en SEQ ID NOs: 19-21, et 54-57,
et
(b) un acide nucléique qui présente vis-à-vis de l'acide nucléique sous (a) une identité de séquence d'au moins 98%.

3. Utilisation selon la revendication 1 ou 2, dans laquelle l'anticorps qui se lie à un antigène associé à une tumeur est un anticorps ou un fragment d'un anticorps monoclonal, chimère ou humanisé.

4. Utilisation selon l'une des revendications 1-3, dans laquelle l'antigène associé à une tumeur comporte une séquence d'acides aminés qui est choisie dans le groupe consistant en SEQ ID NOs: 22-24, 58-61, 81 et 82.

5. Anticorps qui se lie de manière spécifique à un antigène associé à une tumeur, dans lequel l'antigène associé à une tumeur est codé par un acide nucléique qui est choisi dans le groupe consistant en:
(a) un acide nucléique qui comporte une séquence d'acide nucléique qui est choisie dans le groupe consistant en SEQ ID NOs: 19-21, et 54-57,
et
(b) un acide nucléique qui présente vis-à-vis de l'acide nucléique sous (a) une identité de séquence d'au moins 98%,
pour utilisation dans un procédé pour le diagnostic d'une affection cancéreuse qui se distingue par l'expression de l'antigène associé à une tumeur.

6. Anticorps selon la revendication 5, dans lequel la protéine ou le polypeptide comporte une séquence d'acides aminés qui est choisie dans le groupe consistant en SEQ ID NOs: 22-24, 58-61, 81 et 82.

7. Anticorps selon la revendication 5 ou 6, dans lequel l'anticorps est un anticorps ou un fragment d'un anticorps monoclonal, chimère ou humanisé.

8. Anticorps selon l'une des revendications 5-7, dans lequel l'anticorps est couplé à un moyen de diagnostic.

9. Anticorps selon la revendication 8, dans lequel le moyen de diagnostic est une toxine.

10. Utilisation d'un kit pour le diagnostic d'une affection cancéreuse qui se distingue par l'expression d'un antigène associé à une tumeur, comportant des moyens pour la mise en évidence
(i) de l'acide nucléique qui code pour l'antigène associé à une tumeur,
(ii) de l'antigène associé à une tumeur,
(iii) d'anticorps qui se lient à l'antigène associé à une tumeur, et/ou
(iv) de cellules T, qui sont spécifiques pour un complexe entre l'antigène associé à une tumeur ou une partie de celui-ci qui comprend au moins 6 acides aminés consécutifs, et une molécule de CMH,
tandis que l'antigène associé à une tumeur présente une séquence qui est codée par un acide nucléique qui est choisi dans le groupe consistant en:
(a) un acide nucléique qui comprend une séquence d'acide nucléique qui est choisie dans le groupe consistant en SEQ ID NOs: 19-21 et 54-57,
et
(b) un acide nucléique qui présente vis-à-vis de l'acide nucléique sous (a) une identité de séquence d'au moins 98%.

11. Utilisation selon la revendication 10, dans laquelle les moyens pour la mise en évidence de l'acide nucléique qui code pour l'antigène associé à une tumeur sont des molécules d'acide nucléique pour l'amplification sélective de l'acide nucléique.

12. Utilisation d'un anticorps qui se lie au TPTE et est couplé avec un moyen de diagnostic, pour la préparation d'une composition pharmaceutique pour le diagnostic ou le suivi d'une tumeur donnant des métastases, qui se distingue par l'expression de TPTE, tandis que le TPTE présente une séquence qui est codée par un acide nucléique consistant en:
(a) un acide nucléique qui comprend la séquence d'acide nucléique selon SEQ ID NO: 19,
et
(b) un acide nucléique qui présente vis-à-vis de l'acide nucléique sous (a) une identité de séquence d'au moins 98%.

13. Utilisation selon la revendication 12, dans laquelle l'anticorps est un anticorps ou un fragment d'un anticorps monoclonal, chimère ou humanisé.

14. Procédé *in vitro* pour le diagnostic d'une affection cancéreuse qui se distingue par l'expression d'un antigène associé à une tumeur, comprenant
(i) la mise en évidence d'un acide nucléique qui code pour un antigène associé à une tumeur, et/ou
(ii) la mise en évidence d'un antigène associé à une tumeur, et/ou
(iii) la mise en évidence d'un anticorps contre un antigène associé à une tumeur, et/ou
(iv) la mise en évidence de cellules T cytotoxiques ou auxiliaires, qui sont spécifiques pour un antigène associé à une tumeur ou une partie de celui-ci qui comprend au moins 6 acides aminés consécutifs, dans un échantillon biologique isolé à partir d'un patient, tandis que
l'antigène associé à une tumeur présente une séquence qui est codée par un acide nucléique qui est choisi dans le groupe consistant en:
(a) un acide nucléique qui présente une séquence d'acide nucléique qui est choisie dans le groupe consistant en SEQ ID NOs: 19-21, et 54-57,
et
(b) un acide nucléique qui présente vis-à-vis de l'acide nucléique sous (a) une identité de séquence d'au moins 98%.

15. Procédé selon la revendication 14, dans lequel la mise en évidence comprend
(i) la mise en contact de l'échantillon biologique avec un moyen qui se lie spécifiquement à l'acide nucléique qui code pour l'antigène associé à une tumeur, à l'antigène associé à une tumeur, aux anticorps ou aux cellules T cytotoxiques ou auxiliaires, et
(ii) la mise en évidence de la formation d'un complexe entre le moyen et l'acide nucléique, l'antigène associé à une tumeur, les anticorps ou les cellules T cytotoxiques ou auxiliaires.

16. Procédé selon la revendication 14 ou 15, dans lequel la mise en évidence est comparée avec la mise en évidence dans un échantillon biologique normal comparable.

17. Procédé *in vitro* pour la détermination de la régression, du déroulement ou du déclenchement d'une affection cancéreuse, qui se distingue par l'expression d'un antigène associé à une tumeur, comprenant le suivi d'un échantillon provenant d'un patient qui présente un cancer ou est soupçonné de souffrir d'un cancer, en ce qui concerne un ou plusieurs paramètres choisis dans le groupe consistant en:
(i) la quantité d'un acide nucléique qui code pour l'antigène associé à une tumeur,
(ii) la quantité de l'antigène associé à une tumeur,
(iii) la quantité d'anticorps qui se lient à l'antigène associé à une tumeur, et
(iv) la quantité de cellules T cytolytiques ou exprimant des cytokines, qui sont spécifiques pour un complexe entre l'antigène associé à une tumeur ou une partie de celui-ci qui comprend au moins 6 acides aminés consécutifs, et une molécule de CMH, tandis que l'antigène associé à une tumeur présente une séquence qui est codée par un acide nucléique qui est choisi dans le groupe consistant en:
(a) un acide nucléique qui comporte une séquence d'acide nucléique qui est choisie dans le groupe consistant en SEQ ID NOs: 19-21, et 54-57,
et
(b) un acide nucléique qui présente vis-à-vis de l'acide nucléique sous (a) une identité de séquence d'au moins 98%.

18. Procédé selon la revendication 17, dans lequel le procédé comprend la détermination du ou des paramètres à un premier moment dans un premier échantillon et à un deuxième moment dans un autre échantillon et l'évolution de la maladie est déterminée par une comparaison des deux échantillons.

19. Procédé selon l'une des revendications 14-18, dans lequel la mise en évidence de l'acide nucléique ou le suivi de la quantité de l'acide nucléique s'effectue avec une sonde polynucléotidique qui hybride spécifiquement avec l'acide nucléique.

20. Procédé selon la revendication 19, dans lequel la sonde polynucléotidique possède une séquence de 6-50 nucléotides contigus provenant de l'acide nucléique qui code pour l'antigène associé à une tumeur.

21. Procédé selon l'une des revendications 14-18, dans lequel la mise en évidence de l'acide nucléique ou le suivi de la quantité d'acide nucléique s'effectue par amplification sélective de l'acide nucléique.

22. Procédé selon l'une des revendications 14-18, dans lequel la mise en évidence de l'antigène associé à une tumeur ou le suivi de la quantité d'antigène associé à une tumeur s'effectue avec un anticorps qui se lie spécifiquement à l'antigène associé à une tumeur.

23. Procédé selon l'une des revendications 14-18, dans lequel la mise en évidence de l'anticorps ou le suivi de la quantité d'anticorps s'effectue avec une protéine ou un peptide qui se lie spécifiquement à l'anticorps.

24. Procédé selon l'une des revendications 14-18, dans lequel la mise en évidence des cellules T ou le suivi de la quantité de cellules T s'effectue avec une cellule qui présente le complexe entre l'antigène associé à une tumeur ou une partie de celui-ci et une molécule de CMH.

25. Procédé selon l'une des revendications 19, 20, et 22-24, dans lequel la sonde polynucléotidique, l'anticorps, la protéine ou le peptide ou la cellule sont marqués pour être détectables.

26. Procédé selon la revendication 25, dans lequel le marqueur détectable est un marqueur radioactif ou un marqueur enzymatique.

27. Procédé selon l'une des revendications 14-26, dans lequel l'échantillon comprend un fluide corporel et/ou un tissu corporel.

28. Procédé selon l'une des revendications 14-27, dans lequel l'antigène associé à une tumeur comprend une séquence d'acides aminés qui est choisie dans le groupe consistant en SEQ ID NOs: 22-24, 58-61, 81, et 82.
